# EUROPÄISCHE PATENTSCHRIFT

(11) **EP 1 177 442 B1**
(45) Veröffentlichungstag und Bekanntmachung des Hinweises auf die Patenterteilung: **13.08.2008**
(21) Anmeldenummer: 00918871.5
(22) Anmeldetag: 13.04.2000
(51) Int. Cl.: G01N 33/533, G01N 33/58, C07D 271/12

(54) **BESTIMMUNG DER KOMPLEXEN PHOSPHOLIPID/LIPID-STRUKTUREN MIT HILFE SYNTHETISCHER FLUORESZENZ-MARKIERTER ACYLGLYCERIDE**
DETERMINATION OF COMPLEX PHOSPHOLIPID/LIPID STRUCTURES USING SYNTHETIC FLUORESCENCE-MARKED ACYLGLYCERIDES
DETERMINATION DE STRUCTURES COMPLEXES PHOSPHOLIPIDE/LIPIDE A L'AIDE D'ACYLGLYCERIDES SYNTHETIQUES A MARQUAGE FLUORESCENT

(30) Priorität: 30.04.1999 DE 19919634
(43) Veröffentlichungstag der Anmeldung: 06.02.2002
(73) Patentinhaber: Sanofi-Aventis Deutschland GmbH, 65929 Frankfurt am Main (DE)
(72) Erfinder: MUELLER, Guenter, D-65843 Sulzbach (DE); PETRY, Stefan, D-65929 Frankfurt (DE); JORDAN, Holger, D-61276 Weilrod (DE); KLEINE, Horst, D-65795 Hattersheim (DE); WENZEL, Horst, D-60331 Frankfurt (DE)
(86) Internationale Anmeldenummer: PCT/EP2000/003308
(87) Internationale Veröffentlichungsnummer: WO 2000/067025

(56) Entgegenhaltungen:
- EP-A- 0 253 271
- TING ET AL: "An improved synthesis of 7-nitroben-2-oxa-1,3-diazole analogs of CDP-diacylglycerol and phosphatidylinositol" CHEM. PHYS. LIPIDS, Bd. 60, Nr. 1, 1991, Seiten 83-91, XP000937528
- MULLER G (REPRINT) ET AL: "Analysis of lipid metabolism in adipocytes using a fluorescent fatty acid derivative.1. Insulin stimulation of lipogenesis" BIOCHIMICA ET BIOPHYSICA ACTA-LIPIDS AND LIPID METABOLISM, (12 JUL 1997) VOL. 1347, NO. 1, PP. 23-39. PUBLISHER: ELSEVIER SCIENCE BV, PO BOX 211, 1000 AE AMSTERDAM, NETHERLANDS. ISSN: 0005-2760., XP000937689 HOECHST MARION ROUSSEL, PHARMACEUT RES SITE, DG METAB, BLDG H825, D-65926 FRANKFURT, GERMANY (Reprint)
- SKLAN D ET AL: "ASSOCIATION OF ACYL GLYCERIDE AND RETINYL PALMITATE HYDROLASE ACTIVITIES WITH ZINC AND COPPER METALLO PROTEINS IN A HIGH MOLECULAR WEIGHT LIPID PROTEIN AGGREGATE FRACTION FROM CHICK LIVER CYTOSOL" BIOCHIMICA ET BIOPHYSICA ACTA, Bd. 711, Nr. 3, 1982, Seiten 532-538, XP000937676 ISSN: 0006-3002

## Beschreibung

Die Erfindung betrifft einen einfachen kontinuierlichen Test zur Identifizierung von Strukturen, die die Anordnung von Aromaten zur Charge-Transfer-Komplexen begünstigen, wie z.B. komplexer Phospholipid/Lipidstrukturen (Bilayer, Monolayer, Aggregate, Mizellen), mit Hilfe synthetischer fluoreszenz-markierter Acylglyceride und dessen Anwendung zur Bestimmung der Aktivität von Lipasen/Lipasehemmern.

Lipasen, Phospholipasen und andere lipolytische Enzyme haben große Bedeutung im biotechnologischen und medizinischen Bereich. Bei gewissen Stoffwechselerkrankungen läßt sich erhöhte Lipaseaktivität im Fettgewebe nachweisen, die für die Pathogenese dieser Krankheit mit verantwortlich gemacht wird. Der größte Teil der Energiereserven des Körpers ist in Zellen des Fettgewebes als Fettsäuren der Triglyceride gespeichert. Die wesentlichen durch Insulin bewirkten anabolen Vorgänge umfassen die Stimulation der Aufnahme von Substraten für die Triglyceridsynthese und die Erhöhung der Lipogenese. Ein weiterer wichtiger durch Insulin hervorgerufener Prozeß ist die Hemmung der Lipolyse, der Vorgang durch den katabole Hormone, in erster Linie Katecholamine, die Hydrolyse von Triglyceriden stimulieren und dadurch die Freisetzung von Fettsäuren induzieren. Ein wesentliches Problem, welches mit nicht-insulin-abhängigem Diabetes Mellitus (NIDDM) verknüpft ist, hat seine Ursache in der ungehemmten Lipolyse der Fettzellen, die zu erhöhten Spiegeln an nicht veresterten Fettsäuren im Plasma führt. Nach gegenwärtiger Vorstellung stimulieren die Fettsäuren die Glukoneogenese in der Leber und vermindern die Glukoseutilisation im Skelettmuskel über noch schlecht charakterisierte molekulare Mechanismen. Tatsächlich konnte gezeigt werden, daß die Unterdrückung der Lipolyse in Fettzellen durch Hemmstoffe der Lipolyse, wie Agonisten des Nicotinsäurerezeptors der Fettzelle, sowohl die Fettsäurekonzentrationen im Plasma als auch einen erhöhten Blutzucker in diabetischen Tieren und Patienten senkt. Leider sind diese benefitären Effekte nicht besonders stark ausgeprägt und nur von relativ kurzer Dauer. Dies mag auf einer physiologischen Gegenregulation beruhen, hervorgerufen durch Eingriff in den Regelmechanismus des geschwindigkeitsbestimmenden Enzyms der Lipolyse, der hormon-sensitiven Lipase (HSL). Es gibt gute Gründe anzunehmen, daß die Hemmung der lipolytischen Reaktion zu einer verbesserten Therapie des NIDDM führen wird, wenigstens im Hinblick auf die Unterdrückung der Fettsäurefreisetzung aus den Fettzellen. Die direkte Hemmung der HSL durch geeignete Inhibitoren sollte dabei die offensichtlichen Schwierigkeiten eines Eingriffs in die komplexe Regulation der HSL umgehen.

Die Aktivität lipolytischer Enzyme wird traditionell mit radiometrischen, titrimetrischen, enzymatischen oder fluorimetrischen/photometrischen Methoden untersucht. Radiometrische Assays sind am sensitivsten, aber sie erfordern teure radiomarkierte Substrate, sind diskontinuierlich und setzten die Abtrennung des radiomarkierten Substrats vom radiomarkierten Produkt voraus. Solche Trennungen sind oft lästig und die Vermeidung/Reduzierung von radioaktivem Abfall ist von steigender Bedeutung (relevant vor allem bei einer großen Zahl von Testen).
Titrimetrische Teste sind kontinuierlich und können sowohl mit natürlichen- als auch synthetischen Substraten durchgeführt werden, sie leiden aber häufig unter einer recht geringen Sensitivität und sind anfällig gegenüber Bedingungen, die die Menge an freigesetzten Protonen beeinflußen.
Enzymatische oder chromatographische Methoden zum Nachweis eines der Produkte der lipolytischen Reaktion (z.B. Glyzerin) sind sehr empfindlich und relativ robust, aber auch umständlich in der Handhabung, da sie vor dem eigentlichen enzymatischen/chromatographischen Nachweis die Aufarbeitung des Inkubationsansatzes der Lipasereaktion voraussetzen. Die Kopplung an einen Enzymtest erlaubt nur Endpunktmessungen ("time-stop" Messung). Ferner kann bei der Untersuchung unbekannter Substanzen (z. B. Suche nach potentiellen Inhibitoren) eine Wirkung auf die Enzyme der Nachweisreaktion nicht prinzipiell ausgeschlossen werden und erfordert deshalb entsprechende Kontrollen.
Diese Überlegungen gaben den Anstoß zur Entwicklung von fluorimetrischen/photometrischen Verfahren. Diese erreichen prinzipiell die Sensitivität von radiometrischen Methoden, erfordern aber den Einsatz von synthetischen mit Fluorophoren oder Chromophoren modifizierten Substraten oder Proben. Traditionelle fluorimetrische/photometrische Methoden sind wie die radiometrischen Prozeduren diskontinuierlich im Ablauf und bedingen die Abtrennung des Substrats vom Produkt. In jüngerer Zeit wurden kontinuierliche fluorimetrische/photometrische Assays entwickelt (S. Hendrickson, Analyt. Biochem 219 (1994) 1-8), die auf einer Verschiebung des Fluoreszenz- bzw.
Extinktionsmaximums des Produkts im Vergleich zum Substrat basieren. Jedoch beschränken sich alle diese Verfahren auf den Nachweis von Phospholipasen, Lipoproteinlipase, Cholesterinesterase, Sphingomyelinase und Glukosyl-Ceramid-Glukosidase. Substrate mit fluorophoren/chromophoren Gruppen, geeignet für die kontinuierliche Aktivitätsmessung von Triglyzerid-spaltenden Enzymen (z.B. hormon-sensitive Lipase, Monoglyceridlipase, Diglyceridlipase, Triglyceridlipase, Lipoproteinlipase, pankreatischen Lipase, hepatischen Lipase, bakteriellen Lipase, PLA₂, PLC, Cholesterinesterase) sind bisher nicht bekannt.

Ziel der Erfindung ist es daher, einen einfachen kontinuierlichen Test zur Identifizierung von Strukturen, die die Anordnung von Aromaten zur Charge-Transfer-Komplexen begünstigen, wie z.B. komplexer Phospholipid/Lipid-Strukturen (Bilayer, Monolayer, Aggregate, Mizellen), mit Hilfe synthetischer fluoreszenz-markierter Acylglyceride zu entwickeln sowie ein Verfahren zur Bestimmung der Aktivität von lipidbindenden Proteinen, wie Lipasen.

Lipidtransporter sind Proteine, die Lipide erkennen und nicht wie Lipasen spalten, sondern stattdessen durch biologische Membranen transportieren.
Unter Lipasen sind hier biologisch relevante körpereigene Lipasen zu verstehen, wie sie z.B. in R. D. Schmid, R. Verger, Angew. Chem. 110 (1998) 1694-1720 definiert sind.

Unter einem hormonsensitiven Enzym versteht man ein Enzym, das von sekundären Botenstoffen (z.B. cAMP) abhängiger Phosphorylierung oder über andere allosterische Mechanismen (z.B. Protein-Protein Wechselwirkung), die unter Hormonkontrolle stehen, in seiner Aktivität beeinflußt wird. Hormone, die den c-AMP-Spiegel regulieren, sind beispielsweise Adrenalin, Noradrenalin, Glucagon und Insulin.

Gegenstand der Erfindung ist ein Verfahren zur Herstellung eines Substrats, das dadurch gekennzeichnet ist, daß man
a) eine mit einem Fluoreszenzlabel versehene Fettsäure mit 2,3-Epoxypropanol zu einem Monoacylglycerid in alkoholischer Lösung, bei Raumtemperatur unter Zusatz einer Base, wie beispielsweise einer nicht nucleophilen anorganischen Base, umsetzt,
b) dieses Monoacylglycerid mit Phospholipiden im Verhältnis 1:10 bis 10:1 mg/mL einer Ultraschallbehandlung unterzieht, woraus das Substrat resultiert, das an einem Farbumschlag von gelb nach rot erkennbar ist.

Ein Fluoreszenzlabel wird definiert als eine chemische Gruppe innerhalb eines Moleküls, die nach Anregung durch Licht selbst zur Lichtemission befähigt ist. Solche Gruppen werden hier eingesetzt, um Substanzen herzustellen, die selbst in geringsten Konzentrationen von ca. 1 nM noch nachweisbar sind. Erwähnt seien beispielsweise N,N-Dimethylaminosulfonsäure (Dansyl) oder NBD, bevorzugt NBD.

Unter einer Fettsäure versteht man beispielsweise eine langkettige Carbonsäure, gesättigt oder ungesättigt mit einer Kettenlänge von C-8 bis C-20, bevorzugt C-12, C-14, C-16 und C-18 gesättigt oder ungesättigt, besonders bevorzugt C-12 gesättigt.

Eine mit einem Fluoreszenzlabel versehene Fettsäure wurde mit 2,3-Epoxypropanol zu einem Monoacylglycerid gekoppelt. Aus diesem synthetischen Substrat und Phospholipiden, wie z.B. Phosphatidylinositol und Phosphatidylcholin allein oder gemeinsam, gegebenenfalls in einem Verhältnis nach Gewicht von 10:1 bis 1:10, bevorzugt 3:1 bis 1:3, besonders bevorzugt 2:1, wurden durch Ultraschallbehandlung, die beispielsweise ca. 1 bis 10 Minuten, bevorzugt 1 bis 6 Minuten, besonders bevorzugt 4 Minuten dauert, Micellen oder Vesikel gebildet, die als Substrat der zu untersuchenden Lipase dienen. Dieser Einbau in Micellen oder Vesikel ist mit einem Farbumschlag von gelb nach rot verbunden, beruhend auf einem Charge-Transfer-Komplex der in dieser Struktur räumlich eng benachbarten Aromaten. Inkubation mit Lipase führt zur Abspaltung der Fettsäuren unter Freisetzung von markierter Fettsäure und Glycerin.

Als Phospholipide werden beispielsweise Phosphatidylcholin (6 mg) und Phosphatidylinositol (6 mg) in Chloroform (je 1 ml) gelöst. Zur Herstellung des Substrats werden zwei Teile Phosphatidylinositollösung (z.B. 83.5 µl) und ein Teil Phosphatidylcholinlösung (z.B. 41.5 µl) und 100 µl NAG Lösung (10 mg in 1 ml Chloroform) zusammenpipettiert (Endkonzentration im Test: 0.0375 mg Phospholipid/ml; 0.05 mg/ NAG/ml). Nach Entfernen des Chloroform werden 20 ml 25 mM Tris/HCl, pH 7.4; 150 mM NaCl hinzugegeben und zwei Ultraschallbehandlungen mit einem Ultraschallstab (Branson Sonifier Type II, Standardmikrospitze, 25 W) durchgeführt: 1. Behandlung: Einstellung 2, 2 x 1 min, dazwischen jeweils 1 min auf Eis; 2. Behandlung: Einstellung 4, 2 x 1 min, dazwischen jeweils 1 min auf Eis. Während dieser Prozedur verändert sich sich die Farbe der Substratlösung von gelb (Extinktionsmaximum 481 nm) nach rot (Extinktionsmaximum 550 nm) durch Interkalation von NAG zwischen die Phospholipidmoleküle der Vesikel/Micellen.

Die freie Fettsäure bildet keine Micellen oder Vesikel. Deshalb beobachtet man bei der Abspaltung der Fettsäure von den Micellen/Vesikeln einen Farbumschlag von rot nach gelb. Somit wird die Zerstörung der Micellen/Vesikel und damit die Enzymaktivität der Lipase meßbar, entweder visuell (bei 481 nm, bzw. bei 550 nm), über die Farbänderung von rot nach gelb mit Hilfe eines Küvetten-Photometers (z.B. DU-640 der Firma Beckman (München)) oder eines Mikrotiterplattenlesegeräts (z.B. Microßeta der Firma Wallac (Turku, Finnland) oder alternativ fluorimetrisch mit Hilfe eines Phosphoimagers (z.B. Storm 840 der Firma Molecular Dynamics (Krefeld)), eines Fluoreszenzscanners (z.B. DA-2 der Firma Shimadzu (Osaka, Japan)) oder eines Bildauswerteverfahrens (z.B. ArrayScan der Firma Molecular Devices (USA)) welches auf einer CCD-Kamera (charge-coupled device), die einen integrierten Schaltkreis zur Verarbeitung elektrischer und optischer Signale hat, bei dem die Information in Form elektrischer Ladungen gespeichert und weitergeleitet wird, beruht.

Bei einem bevorzugten Verfahren zur Herstellung eines Substrats ist das Fluoreszenzlabel Dansyl oder NBD.
Bei einem besonders bevorzugten Verfahren zur Herstellung eines Substrats ist das Fluoreszenzlabel NBD.

Bei einem weiteren bevorzugten Verfahren zur Herstellung eines Substrats ist die Fettsäure eine gesättigte oder ungesättigte Carbonsäure mit einer Kettenlänge von C-8 bis C-20.
Bei einem weiteren besonders bevorzugten Verfahren zur Herstellung eines Substrats ist die Fettsäure eine gesättigte C-12-Carbonsäure.

Bei einem weiteren bevorzugten Verfahren zur Herstellung eines Substrats werden als Phospholipide Phosphatidylcholin und Phosphatidylinositol allein oder gemeinsam eingesetzt.
Bei einem weiteren besonders bevorzugten Verfahren zur Herstellung eines Substrats werden als Phospholipide Phosphatidylcholin und Phosphatidylinositol gemeinsam eingesetzt im Verhältnis 10 : 1 bis 1 : 10.

Bei einem weiteren bevorzugten Verfahren zur Herstellung eines Substrats ist das Monoacylglycerid 12-(7-Nitro-benzo[1,2,3]oxadiazol-4-ylamino)-dodecansäure-2,3-dihydroxypropylester.

Alle diese Methoden werden bevorzugt in Verbindung mit einem Hochdurchsatzscreening (HTS) eingesetzt. Weitere Verfahren, die auf diesem Konzept beruhen, sind die Messung der Cytotoxizität von Verbindungen und der Wirkung von Detergentien.

Die Erfindung betrifft auch ein Substrat, hergestellt nach dem oben beschriebenen Verfahren und ein Substrat zur Anwendung in einem Verfahren zur Identifizierung von Phospholipid-/Lipidstrukturen, besonders bevorzugt von Lipasen/Lipasehemmern, wie oben beschrieben. Das Verfahren kann auch zur Zerstörung von Mono- oder Bilayerstrukturen, gekrümmt (z.B. Micellen oder Vesikel) oder planar (z.B. künstlich erzeugter gerader Bilayer) eingesetzt werden, die durch eine Farbänderung begleitet ist. Die Farbänderung kann visuell/optisch bzw. fluorimetrisch meßbar verfolgt werden, wie oben beschrieben.

Gegenstand der Erfindung ist ferner ein Verfahren zur Herstellung des Monoacylglycerids 12-(7-Nitro-benzo[1,2,3]oxadiazol-4-ylamino)-dodecansäure-2,3-dihydroxypropylester, wobei man 12-Aminolaurinsäure zunächst mit 7-Chlor-4-nitrobenzo-2-oxa-1,3-diazol und anschließend das erhaltene Zwischenprodukt mit 2,3-Epoxypropanol in alkoholischer Lösung bei Raumtemperatur unter Zusatz einer Base umsetzt, sowie das Monoacylglycerid 12-(7-Nitro-benzo[1,2,3]oxadiazol-4-ylamino)-dodecansäure-2,3-dihydroxypropylester selbst.

Die Aufgabe der Erfindung wird gelöst durch ein Verfahren zur Identifizierung von Lipasen/Lipasehemmern, deren Vorhandensein eine Farbänderung hervorruft, dadurch gekennzeichnet, daß man
a) ein Substrat wie oben beschrieben bereitstellt,
b) dieses Substrat mit einer Lipase inkubiert, und
c) die Farbänderung bestimmt.
Ebenfalls Gegenstand der Erfindung ist ein Verfahren zur Bestimmung der Aktivität von Lipasen/Lipasehemmern, dadurch gekennzeichnet, daß man
a) ein Substrat wie oben beschrieben bereitstellt,
b) dieses Substrat mit einer Lipase inkubiert,
c) die Geschwindigkeit der Farbänderung bestimmt und
d) die Aktivität ermittelt.

Bei einem bevorzugtes Verfahren zur Bestimmung der Aktivität von Lipasen/Lipasehemmern wird das Substrat mit einer hormon-sensitiven Lipase inkubiert.

Eine typische Reaktion wird beispielsweise in 1.5-ml Eppendorfgefäßen oder 96-Lochplatten für 60 min bei 30°C durchgeführt. 10 µl einer Testsubstanz (z. B. Inhibitoren der HSL) werden in Assaypuffer (25 mM Tris/HCl, pH 7.4; 150 mM NaCl) in Gegenwart von 16.6 % DMSO vorgelegt. 180 µl der Substratlösung (20 µg/ml Phosphatidylcholin, 10 µg/ml Phosphatidylinositol, 50 µg/ml NAG in Assaypuffer) werden hinzugegeben. Nach einer Vorinkubation für 15 min bei 30°C werden 20 µl HSL in Assaypuffer hinzupipettiert und die Extinktion bei 481 nm in einem Küvettenphotometer (0.5 ml-Küvette) bzw. Mikrotiterplattenlesegerät sofort gemessen (s.o.). Nach einer gewissen Inkubationszeit, die variabel ist und von der gewählten Enzymkonzentration abhängt und zwischen 2 und 240 Minuten betragen kann, hier 60 min Inkubation bei 30°C wird die Extinktion erneut gemessen. Die Zunahme der Extinktion im Gelb-Bereich, hier bei 481 nm ist ein Maß für die Enzymaktivität.

Assay Systeme zur Identifizierung eines Lipasehemmers bzw. zur Bestimmung der Aktivität einer Lipase/eines Lipasehemmers gehören ebenfalls zum Gegenstand der Erfindung. Sie enthalten ein Substrat wie oben beschrieben, eine Ultraschallvorrichtung und gegebenenfalls eine Vorrichtung zur visuellen/optischen und/oder fluorimetrischen Bestimmung der Farbänderung von rot nach gelb bzw. neben einem Substrat wie oben beschrieben und einer Ultraschallvorrichtung eine Vorrichtung zur Bestimmung der Geschwindigkeit der Farbänderung und eine Vorrichtung zur Absorptions- oder Fluoreszenzmessung.

Das Assay System kann auch in Form eines Kits vorliegen, wobei der Assay ein Lipase-Assay ist.

Das Kit enthält ein Substrat wie oben beschrieben gegebenenfalls in einem Assaypuffer und ein Behältnis zur Durchführung des Tests, wie z.B. ein Eppendorfgefäß oder eine Mikrotiterplatte, bevorzugt eine Mikrotiterplatte.

Weitere Gegenstände der Erfindung betreffen Verfahren zur Bestimmung von molekularen Transport/Transfersystemen, zur Messung der Detergenzwirkung von Verbindungen oder zur Untersuchung der Cytotoxizität von Verbindungen (Medikamenten u.ä.) enthaltend ein Substrat wie oben beschrieben und eine Phospholipase, beispielsweise Phospholipasen A₂ (aus Schlangengift) oder C (Bacillus cereus).

Unter Transportem/Transferproteine versteht man Proteine, die zentrale Nährstoffe wie Kohlenhydrate, Lipide, und Proteine selbst erkennen und durch biologische Membranen transportieren bzw. von einer bestimmten biologischen Membran zu einer anderen transferieren. Transporter/Transferproteine, beispielsweise isoliert aus Rattenileum, werden in Phospholipidvesikel (Liposomen) funktionell rekonstituiert (Proteoliposomen) bzw. als lösliche Polypeptide zusammen mit Liposomen inkubiert und dann zu einer wie oben beschriebenen Mischung aus Phospholipiden und NBD-Glycerid gegeben und mit Ultraschall behandelt. Der Transportvorgang bzw. Transfervorgang des NBD-Glycerids von den NBD-Glycerid enthaltenden Micellen/Vesikeln in das Lumen der Proteoliposomen mit Hilfe der Transporter bzw. in die Membran der Liposomen mit Hilfe der Transferproteine führt zur Auflösung/Zerstörung der Micellenstruktur und kann wiederum photometrisch oder fluorimetrisch wie oben beschrieben verfolgt werden.

Die Detergenzwirkung von chemischen Verbindungen beruht auf der direkten Zerstörung der Micellen/Vesikel. Da auch biologische Membranen derart aufgebaut sind, sind solche Verbindungen meist cytotoxisch.

Ein Verfahren zur Bestimmung der Detergenzwirkung oder der Cytotoxizität von Verbindungen zeichnet sich dadurch aus, dass man
a) ein Substrat wie oben beschrieben bereitstellt,
b) dieses Substrat mit einer Testverbindung inkubiert, und
c) die Farbänderung von rot nach gelb, die cytotoxische Verbindungen und Verbindungen mit Detergenzwirkung kennzeichnen, visuell/optisch oder fluorimetrisch bestimmt.

Ein Verfahren zur Bestimmung der Aktivität von Lipidtransportern zeichnet sich dadurch aus, dass man
a) ein Substrat wie oben beschrieben bereitstellt,
b) Transporter/Transferproteine in Liposomen funktionell rekonstruiert und
c) die nach b) hergestellten Liposomen zu dem Substrat nach a) gibt und die Farbänderung visuell oder optisch bestimmt.

### Synthesen:

Einige NBD-markierte Fettsäuren wie auch 12-(7-Nitro-benzo[1,2,3]oxadiazol-4-ylamino)-dodecansäure (1) sind zwar kommerziell erhältlich, aber teuer. Obwohl erste Experimente noch mit kommerziell erhältlichem Material durchgeführt wurden, konnte Verbindung (1) in guten Ausbeuten durch Umsetzung von 12-Aminolaurinsäure mit 4-Chloro-7-nitro-benzo[1,2,5]oxadiazol in MeOH erhalten werden.

### Liste der Verbindungen 1 - 9:

| | | |
|---|---|---|
| 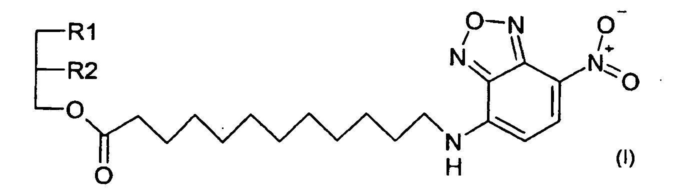 | | |
| 1 | 12-(7-Nitro-benzo[1,2,3]oxadiazol-4-ylamino)-dodecansäure | |
| 2 | 2,3-Epoxypropanol | |
| 3 | R₁ = OH | R₂ = OH |
| 4 | R₁ = OAc | R₂ = OAc |
| 5 | R₁ = OOC(CH₂)₃CH₃ | R₂ = OOC(CH₂)₃CH₃ |
| 6 | R₁ = OOC(CH₂)₁₄CH₃ | R₂ = OH |
| 7 | R₁ = OOC(CH₂)₁₄CH₃ | R₂ = OAc |
| 8 | R₁ = O(CH₂)₁₇CH₃ | R₂ = O(CH₂)₁₇CH₃ |
| 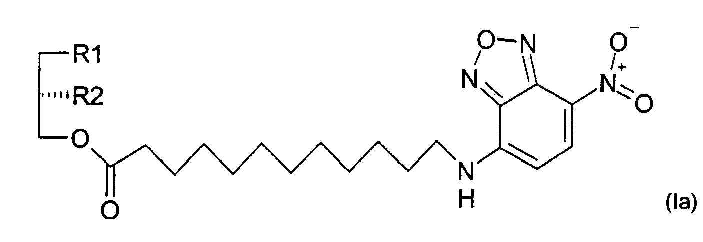 | | |
| 9a | R₁ , R₂ = Isopropyliden | |
| 3a | R₁ , R₂ = OH | |
| 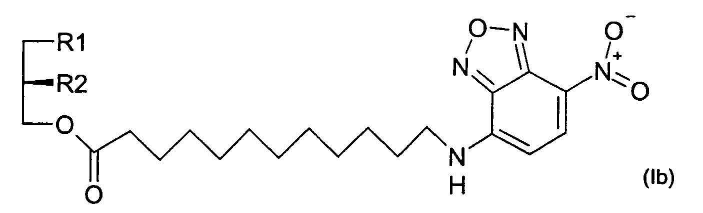 | | |
| 9b | R₁ , R₂ = Isopropyliden | |
| 3b | R₁ , R₂ = OH | |

Durch nucleophile Addition von (1) an 2,3-Epoxypropanol (2) konnte das Monoacylglycerid (3) in guten Ausbeuten erhalten werden. Verbindung (3) wurde anschließend acyliert um zu den Triglyceriden (4) und (5) zu gelangen. Durch Veresterung von (1) mit Palmitin wurde das Diacylglycerid (6) erhalten. Auch diese Verbindung wurde zum entsprechenden Triacylglycerid (7) umgesetzt. Die gleiche Methode wurde benutzt, um den Glycerindiether (8) zum Pseudotriacylglycerid (9) umzusetzen, in dem zwei Acylreste durch langkettige Ether ersetzt sind.

Alle synthetisierten Verbindungen erwiesen sich als Substrate der Lipasen, bevorzugt der HSL, zeigten aber erhebliche Aktivitätsunterschiede. Als "bestes" Substrat der Lipasen erwies sich das Monoacylglycerid (3). Die Einführung weiterer Acylgrupppen, wie in den Verbindungen (4), (5), (6) und (7), führte zu einer Abnahme der Aktivität.

Dies kann leicht mit einer Kompetition der Abspaltung der NBD-Acylgruppe durch die neu eingeführten Acylreste erklärt werden. Um diese These zu erhärten, wurde ein Pseudotriacylglycerid (9) synthetisiert, in dem zwei Acylgruppen durch Hexadecylethereinheiten ersetzt sind. Diese können von den Lipasen nicht abgespalten werden und sollten deshalb nicht mit dem NBD-Fettsäureester kompetitieren. In biologischen Tests erwies sich diese Verbindung zwar als Substrat, allerdings mit geringer Aktivität. Offensichtlich besitzen die Lipasen neben dem katalytischen Bereich einen ausgedehnten für die langkettigen Ethergruppen zugänglichen hydrophoben Bindebereich, so daß die Anlagerung der Fettsäureeinheit behindert wird.

Da sich das Monoacylglycerid (3) als gutes Substrat der Lipasen erwies, wurde untersucht, ob es eine regioselektive Bevorzugung der Position 1 oder 3 gibt. Für diese Untersuchungen wurden die enantiomeren Regioisomeren (3a) und (3b) synthetisiert.

Die Synthese der Enantiomere (3a,b) geht aus von D- und L-1,2-O-Isopropyliden-glycerin, das unter Dicyclohexylcarbodiimid-Aktivierung mit der NBD-markierten Fettsäure (1) verestert wird. Die Abspaltung der Schutzgruppe erfolgte mit 1 N methanolischer HCl. Beide Verbindungen zeigten gleiche biologische Aktivität, so daß der Einsatz enantiomerenreiner Verbindung keinen Vorteil verspricht.

### 1. 12-(7-Nitro-benzo[1,2,3]oxadiazol-4-ylamino)-dodecansäure (1):

Zu einer Lösung von 12-Aminolaurinsäure (18 g, 83.7 mmol) in MeOH (300 ml) wird unter Rühren 30% ige Natriummethanolatlösung (14. 5 ml, 76 mmol) gegeben. Nach 5 Minuten wird die Reaktionsmischung klar und man gibt eine Lösung von 7-Chlor-4-nitrobenzo-2-oxa-1,3-diazol (15 g, 75 mmol) in MeOH (300 ml) zu. Man rührt die Reaktionsmischung, die sofort dunkel wird, für 18 h bei 25° C. Anschließend wird 1 M methanolische HCl (100 ml, 100 mmol) zugegeben und das Lösungsmittel im Vakuum abdestilliert. Der Rückstand wird in MeOH aufgenommen, über Kieselgel filtriert, das Filtrat zur Trockne eingeengt und der Rückstand durch Flashchromatographie (1 : 1 Toluol-EtOAc) gereinigt. Man erhält (1) als roten Feststoff (26.4 g, 93%);
R_{F}: 0.16 (1 : 1 Toluol-EtOAc).
¹H-NMR (250 MHz, CDCl₃): δ 8.5 (d, 1 H, ArH), 6.35 (m, 1 H, NH), 6.18 (d, 1 H, ArH), 3.48 (dt, 2 H, CH₂NH₂), 2.36 (t, 2 H, CH₂COOH), 1,9-1,2 (m, 18 H, 9 CH₂). MS (ESI-MS) 379,2 (M+1).

### 2. 12-(7-Nitro-benzo[1,2,3]oxadiazol-4-ylamino)-dodecansäure-2,3-dihydroxypropylester (3):

Eine Lösung von Verbindung (1) (12 g, 31.7 mmol) und 2,3-Epoxypropanol (50 ml) in Isopropanol (50 ml) wird für 16 h bei 50° C gerührt. Das Lösungsmittel wird im Vakuum abdestilliert, der Rückstand bei 0.01 Torr getrocknet und durch Flashchromatographie (Diisopropylether, Ether, EtOAc) gereinigt. Man erhält Verbindung (3) als rotes Öl (10.3 g, 71.8%);
R_{F}: 0.18 (1:1 Toluol-EtOAc); R_{F}: 0.5 (30:5:1 CH₂Cl₂-MeOH-NH₃), das aus EtOAc-Diethylether kristallisiert.
¹H-NMR (250 MHz, CDCl₃): δ 8.5 (d, 1 H, ArH), 6.35 (m, 1 H, NH), 6.18 (d, 1 H, ArH), 4.19 (dd, 2 H, H-1, H-1'), 3.94 (m, 1 H, H-2), 3.65 (dd, 2 H, H-3, H-3'), 3.48 (dt, 2 H, CH₂NH₂), 2.35 (t, 2 H, CH₂COOH), 1,8 (m, 2 H, CH₂), 1.6 (m, 2 H, CH₂), 1.27-1.15 (m, 14 H, 7 CH₂). MS (ESI-MS) 453.4 (M+1).

### 3. 12-(7-Nitro-benzo[1,2,5]oxadiazol-4-ylamino)-dodecansäure-(S)-2,2-dimethyl-[1,3]dioxolan-4-ylmethylester (9a):

Eine Lösung von Verbindung (1) (60 mg, 159 µmol) in CH₂Cl₂ (2 ml) wird mit Dicyclohexylcarbodiimid (160 mg, 770 µmol) versetzt und 30 min bei 25° C gerührt. Anschließend gibt man eine Lösung von (R)-(2,2-Dimethyl-[1,3]dioxolan-4-yl)-methanol (100 mg, 760 µmol) und Dimethylaminopyridin (94 mg, 770 µmol) in CH₂Cl₂ (2 ml) zu und rührt die Reaktionslösung weitere 4 h bei 25° C. Das Lösungsmittel wird im Vakuum abdestilliert und der Rückstand durch Flashchromatographie (15: 1 Toluol-EtOAc) gereinigt. Verbindung (9a) wird als gelb fluoreszierendes Öl erhalten (46 mg, 58%).
R_{F} 0.29 (4:1 Toluol-EtOAc).
¹H-NMR (CDCl₃): δ 8.5 (d, 1 H, aromat.), 6.2 (m, 1 H, NH), 6.16 (d, 1 H, aromat.), 4.31 (m, 1 H, ), 4.1 (m, 3 H), 3.73 (dd, 1 H ), 3.48 (dt, 2 H, CH₂NH₂), 2.35 (t, 2 H, CO-CH₂), 2.0-1.2 (m, 18 H, 9 CH₂), 1. 42 (s, 3 H, CMe₂), 1.37 (s, 3 H, CMe₂).

### 4. 12-(7-Nitro-benzo[1,2,5]oxadiazol-4-ylamino)-dodecansäure-(R)- 2,2-dimethyl-[1,3]dioxolan-4-ylmethylester (9b):

Verbindung (9b) wird wie für Verbindung (9a) beschrieben hergestellt. Verbindung (9b) wird als gelb fluoreszierendes Öl (51.4 mg, 65%) erhalten.
R_{F} 0.29 (4:1 Toluol-EtOAc).
¹H-NMR (CDCl₃): δ 8.5 (d, 1 H, ArH), 6.2 (m, 1 H, NH), 6.16 (d, 1 H, ArH), 4.31 (m, 1 H, ), 4.1 (m, 3 H), 3.73 (dd, 1 H ), 3.48 (dt, 2 H, CH₂NH₂), 2.32 (t, 2 H, CO-CH₂), 2.0-1.2 (m, 18 H, 9 CH₂), 1. 42 (s, 3 H, CMe₂), 1.37 (s, 3 H, CMe₂).

### 5. 12-(7-Nitro-benzo[1,2,5]oxadiazol-4-ylamino)-dodecansäure-(S)-2,3-dihydroxypropylester (3a) und 12-(7-Nitro-benzo[1,2,5]oxadiazol-4-ylamino)-dodecansäure-(R)-2,3-dihydroxypropylester (3b):

Zu einer Lösung von 13.9 mg ( 28.2 µmol) Verbindung (9a) bzw. 17.8 mg (36.1 µmol) Verbindung (9b) in 25 ml Methanol gibt man methanolische HCl (1 M, 200 µl) und rührt 1.5 h bei 25° C. Das Lösungsmittel wird im Vakuum abdestilliert und der Rückstand durch Flashchromatographie (2:1, 1:1 Toluol-EtOAc) gereinigt. Man erhält die Fettsäureester (3a) und (3b) in einer Ausbeute von 10.5 mg (82%), bzw. 9.3 mg (57%).
¹H-NMR (CDCl₃)-Daten und Massenspektren sind identisch mit Verbindung (3).

### 6. Essigsäure-2-acetoxy-3-[12-(7-nitro-benzo[1,2,5]oxadiazol-4-ylamino)- dode-canoyloxy]-propylester (4):

Verbindung (3) (12 mg, 26.5 µmol) wird in 2:1 Pyridin/Acetanhydrid (3 ml) acetyliert. Nach 8 h engt man zur Trockne ein und reinigt den Rückstand durch Flashchromatographie (1:2 Toluol-EtOAc). Man erhält Verbindung (4) als gelb fluoreszierendes Öl erhalten (13 mg, 91%).
R_{F} 0.66 (1:1 Toluol-EtOAc).
¹H-NMR (250 MHz, CDCl₃): δ 8.5 (d, 1 H, ArH), 6.3 (m, 1 H, NH), 6.18 (d, 1 H, ArH), 5,24 (m, 1 H), 4,92 (m, 1 H), 4,34 (m) 4,28, 4,16, 3,48 (dt, 2 H, CH₂NH₂), 2,32 (CH₂COO), 2,1 (2 s, 6 H, 2 OAc) 2,0-1,0 (m, 18 H, 9 CH₂). MS (ESI-MS): 537,4 (M+1).

### 7. Hexansäure-2-hexanoyloxy-3-[12-(7-nitro-benzo[1,2,5]oxadiazol-4-ylamino)-dodecanoyloxy]-propyl ester (5):

Zu einer Lösung von Verbindung (2) (5 mg, 11 µmol) in Pyridin (300 µl) gibt man Hexansäureanhydrid (100 µl) und läßt die Reaktionsmischung 16 h bei 25° C stehen. Das Lösungsmittel wird im Vakuum abdestilliert und der Rückstand durch Flashchromatographie (9:1 Toluol-EtOAc) gereinigt. Man erhält Verbindung (5) als gelb fluoreszierendes Öl erhalten (1.8 mg, 25%).
R_{F}: 0.73 (1:1 Toluol-EtOAc).
¹H-NMR (250 MHz, CDCl₃): δ. 8.5 (d, 1 H ArH), 6.25 (m, 1 H, NH), 6.18 (d, 1 H, ArH), 5.26 (m, 1 H, H-2), 4,29 (dd, 2 H, H-3, H-3'), 4.15 (dd, 2 H, H-1, H-1'), 3.46 (dt, 2H, CH₂-NH), 2,34 (t, 6 H, 3 CH₂COO), 1,63-1.2 (m, 12 H, 6 CH₂), 0.88 (m, 6 H, 2 CH₃).
MS (ESI-MS): 649.5 (M+1).

### 8. Hexadecansäure-2-hydroxy-3-[12-(7-nitro-benzo[1,2,5]oxadiazol-4-ylamino)-dodecanoyloxy]-propyl ester (6):

Eine Lösung von Verbindung (1) (25 mg, 66 µmol), 4-Dimethylaminopyridin (9 mg, 73 µmol) und 1,1-Carbonyldiimidazol (15 mg, 73 µmol) in CH₂Cl₂ (5 ml) wird 30 min bei 25° C gerührt. Das Lösungsmittel wird im Vakuum abdestilliert und der Rückstand durch Flashchromatographie (9:1 Toluol-EtOAc) gereinigt. Man erhält Verbindung (6) als gelb fluoreszierendes Öl (9.5 mg, 21 %).
R_{F} 0.25 (5:1 Toluol-EtOAc).
¹H-NMR (250 MHz, CDCl₃):δ. 8.54 (d, 1 H ArH), 6.3 (m, 1 H, NH), 6.19 (d, 1 H, ArH), 4.20 (dd, 2 H, H-1, H-1'), 4.15 (dd, 2 H, H-3, H-3'), 4.12 (m, 1 H, H-2), 3.5 (dt, 2H, CH₂-NH), 2,36 (t, 4 H, 2 CH₂COO), 1.82 (m, 2 H, CH₂-CH₂-NH), 1,63 (m, 4 H, 2 CH₂CH₂COO), 1.47 (m, 2 H, CH₂-(CH₂)₂-NH), 1.31-1.26 (m, 22 H, 11 CH₂), 1.287 (m, 2 H, CH₂), 1.26 (m, 2 H, CH₂), (m, 4 H, 2 CH₂CH₂CH₂COO), 1.31-1.26 (m, 18 H, 9 CH₂), 1.3 (m, 2 H, CH₂), 1.26 (m, 2 H, CH₂), 0.89 (t, 3 H, CH₃). MS (ESI-MS): 649.5 (M+1).

### 9. Hexadecansäure-2-acetyloxy-3-[12-(7-nitro-benzo[1,2,5]oxadiazol-4-ylamino)- dodecanoyloxy]-propyl ester (7):

Verbindung (6) (6 mg, 8,7 µmol) wird acetyliert und aufgearbeitet, wie für Verbindung (4) beschrieben. Das Rohprodukt wird durch Flashchromatographie (9:1 Toluol-EtOAc) gereinigt. Man erhält Verbindung (7) als gelb fluoreszierendes Öl (5.1 mg, 95%).
R_{F} 0.71 (5:1 Toluol-EtOAc).
¹H-NMR (250 MHz, CDCl₃):δ 8.5 (d, 1 H, ArH), 6.25 (m, 1 H, NH), 6.17 (d, 1 H, ArH), 5.25 (m, 1 H, H-2), 4.27 (dd, 2 H, H-1, H-3), 4.15 (dd, 2 H, H-1', H-3'), 3.73 (dt, 4 H, CH₂-NH), 2.3 (t, 2 H, CH₂COO), 2.08 (s, 3 H, OAc), 1.8 (m, 2 H, CH₂-CH₂-NH), 1.6 (m, 8 H, 4 CH₂), 1.4-1 (m, 32 H, 16 CH₂), 0.85 (t, 3 H, CH₃).
MS (FAB-MS): 739 (M+L1).

### 10. 12-(7-Nitro-benzo[1,2,5]oxadiazol-4-ylamino)-dodecansäure 2,3-bisoctadecyloxy-propyl ester (8):

Zu einer Lösung von Verbindung (1) (3 mg, 7.9 µmol), Dimethylaminopyridin (5 mg, 41 µmol) und Dicyclohexylcarbodiimid (5 mg, 24 µmol) in CH₂Cl₂ gibt man 2,3-Bis-octadecyloxypropan-1-ol (5 mg, 8.37 µmol). Die Reaktionsmischung wird 2 h bei 25° C gerührt, das Lösungsmittel im Vakuum abdestilliert und der Rückstand durch Flashchromatographie (2:1 Petrolether-Diethylether) gereinigt. Man erhält Verbindung (8) als gelb fluoreszierendes Öl (3.5 mg, 46%).
R_{F} 0.55 (3:7 Diethylether/Petrolether). MS (FAB-MS): 957.8 (M+1).

### Enzympräparation:

### Präparation der partiell gereinigten HSL:

Isolierte Rattenfettzellen werden aus Nebenhodenfettgewebe von nicht-behandelten männlichen Ratten (Wistar, 220-250 g) durch Kollagenasebehandlung gemäß publizierter Verfahren gewonnen. Die Fettzellen aus 10 Ratten werden dreimal durch Flotation mit jeweils 50 ml Homogenisationspuffer (25 ml Tris/HCl, pH 7.4, 0.25 M sucrose, 1 mM EDTA, 1 mM DTT, 10 µg/ml Leupeptin, 10 µg/ml Antipain, 20 µg/ml Pepstatin) gewaschen und schließlich in 10 ml Homogenisationspuffer aufgenommen. Die Fettzellen werden im Teflon-in-Glas Homogenisator (Braun-Melsungen) durch 10 Hübe bei 1500 rpm und 15°C homogenisiert. Das Homogenisat wird zentrifugiert (Sorvall SM24-Röhrchen, 5000 rpm, 10 min, 4°C). Der Unterstand zwischen der oben liegenden Fettschicht und dem Pellet wird abgenommen und die Zentrifugation wiederholt. Der daraus resultierende Unterstand wird erneut zentrifugiert (Sorvall SM24-Röhrchen, 20000 rpm, 45 min, 4°C). Der Unterstand wird abgenommen und mit 1 g Heparin-Sepharose (Pharmacia-Biotech, CL-6B, 5 x gewaschen mit 25 mM Tris/HCl, pH 7.4, 150 mM NaCl) versetzt. Nach Inkubation für 60 min bei 4°C (in Intervallen von 15 min aufschütteln) wird der Ansatz zentrifugiert (Sorvall SM24-Röhrchen, 3000 rpm, 10 min, 4°C). Der Überstand wird durch Zugabe von Eisessig auf pH 5.2 gebracht und 30 min bei 4°C inkubiert. Die Präzipitate werden durch Zentrifugation (Sorvall SS34, 12000 rpm, 10 min, 4°C) gesammelt und in 2.5 ml 20 mM Tris/HCl, pH 7.0, 1 mM EDTA, 65 mM NaCl, 13 % sucrose, 1 mM DTT, 10 µg/ml Leupeptin/Pepstatin/Antipain suspendiert. Die Suspension wird über Nacht bei 4°C gegen 25 mM Tris/HCl, pH 7.4, 50 % Glyzerin, 1 mM DTT, 10 µg/ml Leupeptin, Pepstatin, Antipain dialysiert und dann auf eine Hydroxyapatit-Säule aufgetragen (0.1 g pro 1 ml Suspension, äquilibriert mit 10 mM Kaliumphosphat, pH 7.0, 30 % Glyzerin, 1 mM DTT). Die Säule wird mit vier Volumina Äquilibrierungspuffer bei einer Flußrate von 20 bis 30 ml/h gewaschen. Die HSL wird mit einem Volumen Äquilibrierungspuffer, der 0.5 M Kaliumphosphat enthält, eluiert, sodann dialysiert (s.o.) und 5- bis 10-fach konzentriert durch Ultrafiltration (Amicon Diaflo PM 10 Filter) bei 4°C. Die partiell gereinigte HSL kann 4 bis 6 Wochen bei -70°C aufbewahrt werden.

### Präparation des NAG (NBD-Monoacylglycerid) Substrats:

6 mg Phosphatidylcholin und 6 mg Phosphatidylinositol werden in je 1 ml Chloroform gelöst. 10 mg NAG werden in 1 ml Chlorofrom gelöst. Zwei Teile Phosphatidylinositollösung (z.B. 83.5 µl) und ein Teil Phosphatidylcholinlösung (z.B. 41.5 µl) und 100 µl NAG Lösung werden in Plastikszintillationsgefäßen zusammenpipettiert (Endkonzentration im Test: 0.0375 mg Phospholipid/ml; 0.05 mg/ NAG/ml). Das Chloroform (225 µl Gesamtvolumen) wird durch Überblasen mit einem N₂-Strom komplett entfernt. Das getrocknete Substrat kann für bis zu 3 Tagen bei 4°C aufbewahrt werden. Zur Herstellung der Phospholipidvesikel/Micellen mit interkaliertem NAG (am Testtag) wird das getrocknete Substrat in 20 ml Assaypuffer (25 mM Tris/HCl, pH 7.4; 150 mM NaCl) aufgenommen und zwei Ultraschallbehandlungen mit einem Ultraschallstab (Branson Sonifier Type II, Standardmikrospitze): 1. Behandlung Einstellung 2, 2 x 1 min, dazwischen jeweils 1 min auf Eis; 2. Behandlung Einstellung 4, 2 x 1 min, dazwischen jeweils 1 min auf Eis. Während dieser Prozedur verändert sich sich die Farbe der Substratlösung von gelb (Extinktionsmaximum 481 nm) nach rot (Extinktionsmaximum 550 nm) durch Interkalation von NAG zwischen die Phospholipidmoleküle der Vesikel/Micellen. Vor Benutzung als Substrat (innerhalb der nächsten 2 h) wird die Lösung noch 15 min auf Eis inkubiert.

### Indirekter NAG Assay:

Der Assay wird in 1.5-ml Eppendorfgefäßen oder 96-Lochplatten für 60 min bei 30°C durchgeführt. Für das Auffinden von Inhibitoren der HSL werden 10 µl der Testsubstanz in Assaypuffer (25 mM Tris/HCl, pH 7.4; 150 mM NaCl) in Gegenwart von 16.6 % DMSO vorgelegt. 180 µl der Substratlösung (20 µg/ml Phosphatidylcholin, 10 µg/ml Phosphatidylinositol, 50 µg/ml NAG in Assaypuffer) werden hinzugegeben. Nach einer Vorinkubation für 15 min bei 30°C werden 20 µl der Enzymlösung in Assaypuffer (1- bis 4-fach verdünnt) hinzupipettiert und die Extinktion bei 480 nm in einem Küvettenphotometer (0.5 ml-Küvette) bzw. Mikrotiterplattenlesegerät (Microßeta, Wallac) sofort gemessen. Nach 60 min Inkubation bei 30°C wird die Extinktion erneut gemessen. Die Zunahme der Extinktion bei 480 nm ist ein Maß für die Enzymaktivität. Unter Standardbedingungen führen 20 µg partiell gereinigte HSL zu einer Extinktionsänderung von 4000 arb. units.

### Direkter NAG Assay:

Alternativ zur Messung der Extinktionsänderung der Substratlösung werden die Produkte der HSL Reaktion durch Phasentrennung/Dünnschichtchromatographie untersucht. Dazu wird der Inkubationsansatz (200 µl Gesamtvolumen, s. indirekter NAG Assay) in 2 ml Eppendorfgefäßen mit 1.3 ml Methanol/Chloroform/Heptan (10:9:7) und anschließend mit 0.4 ml 0.1 M NaOH versetzt. Nach intensivem Mischen (10 sec) wird die Phasentrennung durch Zentrifugation (800xg, 20 min, Raumtemperatur) eingeleitet. Von der wässrigen oberen Phase werden äquivalente Volumen (z. B. 0.4 ml) abgenommen und die Extinktion photometrisch bei 481 nm bestimmt. Zur Dünnschichtchromatographie wird die wässrige Phase getrocknet (SpeedVac) und dann in 50 µl Tetrahydrofuran aufgenommen. 5-µl Proben werden auf Silicagel Si-60 Platten (Merck, Darmstadt) aufgetragen. Die Chromatographie wird mit 78 ml Diethylether/22 ml Petroläther/1 ml Eisessig als Laufmittel durchgeführt. Die Menge an freigesetzter fluoreszierender NBD-Fettsäure wird durch Phosphorimaging (Molecular Dynamics, Storm 840 und ImageQuant Software) bei einer Anregungswellenlänge von 460 nm und Emissionswellenlänge von 540-560 nm bestimmt.

### TAG Assay:

Für die Herstellung des Substrats werden 25-50 µCi [³H]Trioleoylglycerin (in Toluol), 6.8 µMol unmarkiertes Trioleoylglycerin und 0.6 mg Phospholipide (Phosphatidylcholin/Phosphatidylinositol 3:1 w/v) gemischt, über N₂ getrocknet und dann in 2 ml 0.1 M KPᵢ (pH 7.0) durch Ultraschallbehandlung (Branson 250, Mikrospitze, Einstellung 1-2, 2 x 1 min im 1-min Intervall) aufgenommen. Nach Zugabe von 1 ml KPᵢ und erneuter Ultraschallbehandlung (4 x 30 sec auf Eis in 30-sec Intervallen) wird 1 ml 20% BSA (in KPᵢ) hinzugefügt (Endkonzentration Trioleoylglyzerin 1.7 mM). Für die Reaktion werden 100 µl Substratlösung zu 100 µl HSL-Lösung (HSL präpariert wie oben, verdünnt in 20 mM KPᵢ, pH 7.0, 1 mM EDTA, 1 mM DTT, 0.02% BSA, 20 µg/ml Pepstatin, 10 µg/ml Leupeptin) pipettiert und für 30 min bei 37°C inkubiert. Nach Zugabe von 3.25 ml Methanol/Chloroform/Heptan (10:9:7) und von 1.05 ml 0.1 M K₂CO₃, 0.1 M Borsäure (pH 10.5) wird gut gemischt und schließlich zentrifugiert (800xg, 20 min). Nach Phasentrennung wird ein Äquivalent der oberen Phase (1 ml) abgenommen und die Radioaktivität durch Flüssigszintillationsmessung bestimmt.

### PNPB Assay:

10 µl p-Nitrophenylbutyrat (PNPB)(2 mM in Acetonitril), 890 µl 0.1 M KPᵢ (pH 7.25), 0.9% NaCl, 1 mM DTT und 100 µl HSL (präpariert wie oben, verdünnt in diesem Puffer) werden für 10 min bei 37°C inkubiert. Nach Zugabe von 3.25 ml Methanol/Chloroform/Heptan (10:9:7, w/v) und kräftigem Schütteln wird zentrifugiert (800xg, 20 min) und für 3 min bei 42°C inkubiert. Anschließend wird ein äquivalentes Volumen der oberen Phase abgenommen und die Absorption bei 400 nm bestimmt.

### Tributyrin Assay:

Zur Herstellung des Substrats werden 5 µCi [1-¹⁴C]Tributyrin (in Toluol) zu 1 ml von 20 mM unmarkiertem Tributyrin (in Acetonitril) gegeben. 10 µl dieser Substratlösung werden mit 390 µl 0.1 M KPᵢ (pH 7.25), 0.9 % NaCl, 1 mM DTT, 2 % BSA und 100 µl HSL (präpariert wie oben, verdünnt in diesem Puffer) für 30 min bei 37°C inkubiert. Nach Zugabe von 3.25 ml Methanol/Chloroform/Heptan (10:9:7, w/v) und von 1 ml 0.1 M NaOH wird kräftig gemischt und schließlich zentrifugiert (800xg, 20 min). Ein äquivalentes Volumen (1 ml) der oberen Phase wird abgenommen und die Radioaktivität durch Flüssigszintillationsmessung bestimmt.

### Auswertung:

Substanzen werden üblicherweise in vier unabhängigen Ansätzen geprüft. Die Hemmung der enzymatischen Aktivität der HSL durch eine Testsubstanz wird durch den Vergleich mit einer nicht-gehemmten Kontrollreaktion bestimmt. Die Berechnung des IC₅₀-Wertes erfolgt über eine Hemmkurve mit mind. 10 Konzentrationen der Testsubstanz. Für die Analyse der Daten wird das Softwarepaket GRAPHIT, Elsevier-BIOSOFT (Version 3.0) benutzt.

### Beispiele:

### Beispiel 1: Kinetik der Spaltung von NAG durch HSL

NAG (0.05 mg/ml) wird mit den angegebenen Proteinmengen an partiell gereinigter HSL inkubiert (temperiertes Photometer) und zu bestimmten Zeiten die Extinktion bei 481 nm bestimmt. Zur Inaktivierung wird die HSL für 15 min bei 100°C inkubiert. (n = 8, mean ± SD).
Ergebnis: Bis zu einer Proteinmenge von 20 µg verläuft die Reaktion bis 60 min linear. Der Extinktionsunterschied beträgt hierbei 0.8-0.9 OD. Bei kleineren Proteinmengen ist Linearität bis 180 min gegeben.

### Zunahme der Absorption bei 481 nm (arb. units)

| Zeit [min] | ohne HSL | inaktive HSL | HSL [5 µg] | HSL [10 µg] | HSL [20 µg] | HSL [40 µg] |
|---|---|---|---|---|---|---|
| 0 | 0 | 0 | 0 | 0 | 0 | 0 |
| 1 | 5 ± 2 | 4 ± 1 | 12 ± 2 | 19 ± 4 | 5 ± 7 | 91 ± 14 |
| 2 | 7 ± 2 | 5 ± 2 | 23 ± 4 | 51 ± 9 | 78 ± 12 | 89 ± 32 |
| 5 | 13 ± 4 | 10 ± 4 | 45 ± 9 | 102 ± 19 | 193 ± 36 | 395 ± 62 |
| 10 | 15 ± 3 | 12 ± 3 | 105 ± 18 | 185 ± 26 | 423 ± 62 | 784 ± 88 |
| 15 | 17 ± 5 | 15 ± 4 | 155 ± 20 | 288 ± 39 | 617 ± 70 | 1060 ± 144 |
| 20 | 19 ± 5 | 20 ± 5 | 219 ± 31 | 395 ± 36 | 865 ± 93 | 477 ± 182 |
| 30 | 19 ± 6 | 24 ± 7 | 295 ± 47 | 517 ± 67 | 1143 ± 216 | 2249 ± 286 |
| 45 | 23 ± 6 | 32 ± 6 | 453 ± 52 | 830 ± 57 | 1877 ± 206 | 2968 ± 321 |
| 60 | 25 ± 8 | 37 ± 9 | 677 ± 70 | 1179 ± 110 | 2510 ± 295 | 4065 ± 399 |
| 75 | 28 ± 9 | 39 ± 10 | 831 ± 92 | 1487 ± 104 | 3067 ± 310 | 4834 ± 462 |
| 90 | 31 ± 8 | 41 ± 13 | 995 ± 82 | 1844 ± 130 | 3527 ± 188 | 5472 ± 613 |
| 120 | 35 ± 7 | 45 ± 11 | 1455 ± 102 | 2745 ± 195 | 4941 ± 410 | 5702 ± 531 |
| 180 | 39 ± 11 | 52 ± 15 | 1937 ± 121 | 3521 ± 254 | 5712 ± 399 | 5961 ± 560 |

### Beispiel 2: Abhängigkeit der Spaltung von NAG von der Menge an HSL

NAG (0.05 mg/ml) wird mit den angegebenen Proteinmengen an partiell gereinigter HSL für 60 min inkubiert. In Aliquots der Reaktionsansätze wird die Zunahme der Extinktion bei 481 nm (Entstehung der freien NBD-Fettsäure als Produkt der HSL-Reaktion = indirekter NAG Assay) oder die Abnahme der Extinktion bei 550 nm (Verbrauch von NAG als Substrat der HSL-Reaktion) bestimmt. Alternativ werden weitere Aliquots mit Methanol/Chloroform extrahiert und die in der organischen Phase enthaltene freigesetzte NBD-Fettsäure durch TLC-Analyse und Fluorimetrie (Phosphorlmager Storm 840, Molecular Dynamics) bestimmt (= direkter NAG Assay)(n = 6, mean ± SD).
Ergebnis: Bis zu einer Proteinmenge 20 µg verläuft die Reaktion hinsichtlich der Produktentstehung (Extinktionszunahme bei 481 nm oder Auftreten freier NBD-Fettsäure gemäß TLC-Analyse) als auch hinsichtlich des Substratverbrauchs (Extinktionsabnahme bei 550 nm) linear. Die Übereinstimmung zwischen indirektem und direktem NAG Assay spricht für die Analyse der Spaltung von NAG im indirekten Assay.

### Absorptionsänderung [arb. units]

| HSL Menge [µg] | Zunahme [481 nm] | Abnahme [550 nm] | freigesetzte NBD-FA [550 nm] |
|---|---|---|---|
| 0 | 0 | 885 ± 80 | 0 |
| 1 | 156 ± 21 | 866 ± 94 | 17 ± 2 |
| 2 | 347 ± 40 | 841 ± 78 | 44 ± 5 |
| 5 | 791 ± 88 | 779 ± 82 | 103 ± 11 |
| 7,5 | 1066 ± 120 | 759 ± 69 | 157 ± 21 |
| 10 | 1255 ± 141 | 650 ± 61 | 215 ± 24 |
| 15 | 2351 ± 205 | 540 ± 55 | 320 ± 29 |
| 20 | 2867 ± 199 | 287 ± 51 | 432 ± 53 |
| 25 | 3428 ± 270 | 198 ± 44 | 544 ± 61 |
| 30 | 3973 ± 244 | 115 ± 35 | 616 ± 56 |
| 40 | 4623 ± 511 | 91 ± 19 | 693 ± 67 |
| 60 | 5466 ± 602 | 62 ± 17 | 733 ± 80 |
| 80 | 5913 ± 485 | 54 ± 15 | 746 ± 98 |

### Beispiel 3: Abhängigkeit der Spaltung von NAG durch HSL von der Substratkonzentration

Unterschiedliche Mengen von NAG (bei konstantem Verhältnis zu den Phospholipiden) werden mit den angegebenen Mengen an partiell gereinigter HSL für 60 min inkubiert und dann die Extinktion bei 481 nm bestimmt (n = 5, mean ± SD).
Ergebnis: Die Spaltungsgeschwindigkeit zeigt bei allen drei Enzymmengen den Verlauf einer typischen Sättigungskurve. Aufgrund der enzymatischen Besonderheit der HSL Reaktion ("zweidimensionale" Substratpräsentation, "interfacial activation") läßt sich jedoch nur bei 5 µg Protein und den niedrigsten Substratkonzentrationen ein annähernd lineare Abhängigkeit feststellen. Ein vernünftiger Kompromiß zwischen Substratabhängigkeit und Signalstärke (OD 0.6 bis 0.7) stellt die Kombination von 20 µg Protein und 0.05 mg/ml NAG dar.

### Zunahme der Absorption bei 481 nm (arb. units)

| NAG [mg/ml] | HSL [5 µg] | HSL [20 µg] | HSL [40 µg] |
|---|---|---|---|
| 0,002 | 61 ± 9 | 194 ± 23 | 525 ± 77 |
| 0,005 | 109 ± 17 | 342 ± 58 | 1420 ± 122 |
| 0.01 | 227 ± 31 | 852 ± 77 | 2745 ± 264 |
| 0.02 | 388 ± 45 | 1758 ± 195 | 6705 ± 532 |
| 0.05 | 705 ± 61 | 3002 ± 409 | 11547 ± 912 |
| 0.075 | 1121 ± 107 | 768 ± 502 | 15502 ± 1077 |
| 0.1 | 1521 ± 119 | 6124 ± 577 | 19223 ± 1599 |
| 0.15 | 1983 ± 155 | 7544 ± 603 | 26730 ± 2069 |
| 0.2 | 2242 ± 186 | 8898 ± 961 | 31641 ± 2304 |
| 0.3 | 2489 ± 213 | 9734 ± 825 | 35953 ± 3355 |

### Beispiel 4: Abhängigkeit der Spaltung von NAG durch HSL vom Verhältnis NAG und Phospholipide

NAG (0.05 mg/ml) wird mit unterschiedlichen Mengen an Phospholipiden (Gesamtmenge) unter den angegebenen Verhältnissen von Phosphatidylinositol zu Phosphatidylcholin durch Ultraschallbehandlung als Substrat präpariert und dann mit partiell gereinigter HSL (20 µg) für 60 min inkubiert. Die Zunahme der Extinktion bei 481 nm wird bestimmt (n = 4, mean ± SD).
Ergebnis: Die Enzymgeschwindigkeit ist am größten bei einen PI/PC Verhältnis (nach Gewicht) von 3:1 und 0.0375 bis 0.075 Gesamtphospholipid. Der ausgeprägte Optimumsverlauf für die Gesamtkonzentration der Phospholipide wie auch für deren Zusammensetzung spricht für die Bedeutung der Präsentation der Substrate der HSL (hier NAG) in Phospholipidvesikeln und/oder Micellen bzw. für die Ausbildung eines Monolayers aus Phospholipiden auf dem (neutralen) Core des Substrats.

### Zunahme der Absorption bei 481 nm (arb. units)

| Phosphatidylinositol/ cholin (mg/ml) | PI:PC 3:1 | PI:PC 1:1 | PI:PC 1:3 |
|---|---|---|---|
| 0 | 173 ± 42 | 134 ± 23 | 89 ± 12 |
| 0.009 | 1437 ± 132 | 548 ± 76 | 289 ± 77 |
| 0.018 | 2610 ± 306 | 1430 ± 188 | 692 ± 94 |
| 0.025 | 2980 ± 254 | 1879 ± 205 | 1158 ± 121 |
| 0.0375 | 3144 ± 277 | 1649 ± 170 | 1230 ± 105 |
| 0.075 | 3365 ± 402 | 1138 ± 125 | 1679 ± 143 |
| 0.15 | 2108 ± 256 | 745 ± 97 | 912 ± 103 |
| 0.3 | 913 ± 102 | 335 ± 47 | 427 ± 56 |

### Beispiel 5: Vergleich des indirekten/direkten NAG Assays mit einem konventionellen HSL Assay durch die Bestimmung der IC₅₀-Werte für verschiedene Inhibitoren

NAG (0.05 mg/ml) wird mit 20 µg partiell gereinigter HSL für 60 min in Gegenwart unterschiedlicher Konzentrationen (0.1 bis 100 µM) an verschiedenen Substanzen inkubiert. In Aliquots der Ansätze wird die Extinktion bei 481 nm bestimmt (indirekter Assay) oder die freigesetzte NBD-FA durch Chloroform/Methanol extrahiert und durch TLC Analyse und Fluorimetrie bestimmt (direkter Assay). Alternativ wird [³H]Trioleoylglycerol mit partiell gereinigter HSL gemäß publizierter Bedingungen inkubiert und das freigesetzte radiomarkierte Oleat nach Extraktion mit Chloroform/Methanol durch Flüssigszintillationsmessung bestimmt. Aus den Hemmkurven bestimmt man die IC₅₀-Werte (n = 6, mean ± SD).
Ergebnis: Für alle getesteten Substanzen wurden mit den drei Verfahren typisch sigmoidale Hemmkurven ermittelt. Die IC₅₀-Werte lagen beim indirekten/direkten NAG Assay (Extinktionszunahme bei 481 nm / Freisetzung der NBD-Fettsäure) generell um den Faktor 4 bis 10 niedriger gegenüber der Spaltung von Trioleoylglycerin durch HSL, die Rangfolge der Inhibitoren (gemäß ihrer IC₅₀-Werte) ist jedoch identisch für alle drei Verfahren. Dies bestätigt publizierte Befunde, daß die Wirkung von Inhibitoren der HSL von der Art des Substrats und der Substratpräsentation abhängt. Darüberhinaus können sich auch die effektiven Substratkonzentrationen (NAG und Trioleoylglyzerid) zwischen den beiden Assays unterscheiden (aufgrund der Substratpräparation als Vesikel oder Mizellen kaum bestimmbar) und damit bei kompetitiven Inhibitoren diese Unterschiede erklären. Die nahezu identischen IC₅₀-Werte, die gemäß der Extinktionsänderung und der NBD-Fettsäure-Freisetzung ermittelt wurden, sprechen für Spaltung von NAG durch HSL und damit Freisetzung von NBD-Fettsäure als Ursache für die Extinktionszunahme bei 481 nm, d.h. der NAG Assay erfaßt die lipolytische Spaltung von Lipiden.

### IC₅₀ [µM]

| Compound | NAG-Assay | freigesetzte NBD-FA | TAG-Assay |
|---|---|---|---|
| 1 | 0,78 ± 0,24 | 0,78 ± 0,15 | 5,21 ± 1,44 |
| 2 | 0,75 ± 0,18 | 0,81 ± 0,27 | 12,12 ± 3,20 |
| 3 | 1,52 ± 0,33 | 1,02 ± 0,25 | 15,34 ± 2,43 |
| 4 | 2,45 ± 0,41 | 2,98 ± 0,49 | 18,25 ± 3,54 |
| 5 | 2,79 ± 0,59 | 3,58 ± 0,39 | 18,95 ± 2,31 |
| 6 | 3,42 ± 0,78 | 4,45 ± 0,22 | 21,45 ± 3,06 |
| 7 | 3,82 ± 0,61 | 4,51 ± 0,34 | 33,13 ± 2,88 |
| 8 | 4,29 ± 0,58 | 5,09 ± 0,94 | 35,94 ± 3,18 |
| 9 | 6,37 ± 0,72 | 6,94 ± 0,55 | 39,82 ± 2,95 |
| 10 | 7,51 ± 1,22 | 7,77 ± 1,19 | 47,85 ± 4,12 |
| 11 | 17,94 ± 2,15 | 21,33 ± 2,83 | 78,91 ± 5,66 |
| 12 | 22,38 ± 2,47 | 26,10 ± 3,82 | 116,34 ± 9,42 |

### Beispiel 6: Vergleich des indirekten NAG Assays mit den Assays für TAG, PNPB und Tributyrin

NAG (0.05 mg/ml) wird mit 20 µg partiell gereinigter HSL für 60 min in Gegenwart unterschiedlicher Konzentrationen (0.1 bis 100 µM) an verschiedenen Substanzen inkubiert. In Aliquots der Ansätze wird die Extinktion bei 481 nm bestimmt (indirekter Assay). Alternativ wird [³H]Trioleoylglycerol (TAG), p-Nitrophenylbutyrat (PNPB) oder [¹⁴C]Tributyrin mit partiell gereinigter HSL inkubiert und das freigesetzte radiomarkierte Oleat, *p*-Nitrophenol bzw. Butyrat nach Extraktion mit Chloroform/Methanol durch Flüssigszintillationsmessung bzw. Photometrie bestimmt. Aus den Hemmkurven werden die IC₅₀-Werte bestimmt (n = 5, mean ± SD). Ergebnis: Die relative Rangfolge der Inhibitoren, manifestiert in den IC₅₀-Werten, ist für die beiden Assays mit Lipidsubstrat (NAG und TAG) identisch. Dies gilt grundsätzlich auch für die wasserlöslichen Substrate, Tributyrin und PNPB, doch sind einige Wirkstoffe, die die HSL-Aktivität gegenüber NAG und TAG signifikant reduzieren, unwirksam in der Hemmung der HSL gegenüber Tributyrin und PNPB. Dies läßt sich erklären mit einer Interferenz dieser Hemmstoffe mit der Lipidbindung der HSL (durch die Lipidbindungsdomäne), während der katalytische Mechanismus nicht beeinträchtigt wird. Wasserlösliche Substrate werden deshalb von der HSL auch in Gegenwart dieser Hemmstoffe gespalten. Die IC₅₀-Werte für Inhibitoren, die auch bei wasserlöslichen Substraten wirken, liegen generell zwischen denen für NAG und TAG als Substrat. Dies zeigt, daß sich NAG als "lipidartiges" Substrat für die HSL ähnlich den authentischen Triglyzeriden verhält und der NAG-Assay eingesetzt werden kann zum Auffinden von Hemmstoffen, die die Lipidbindung (wie auch den katalytischen Mechanismus) der HSL blockieren.

### IC₅₀ [µM]

| Compound | NAG-Assay | Tributyrin-Assay | PNPB-Assay | TAG-Assay |
|---|---|---|---|---|
| 1 | 0,85 ± 0,19 | 2,56 ± 0,45 | 3,56 ± 0,67 | 7,45 ± 1,36 |
| 2 | 1,06 ± 0,22 | 3,14 ± 0,38 | 5,03 ± 0,93 | 13,42 ± 2,44 |
| 3 | 2,14 ± 0,29 | > 100 | > 100 | 17,32 ± 1,96 |
| 4 | 3,18 ± 0,51 | > 100 | 93,61 ± 7,92 | 20,45 ± 2,45 |
| 5 | 2,96 ± 0,47 | 4,09 ± 0,59 | 10,45 ± 0,66 | 25,34 ± 3,60 |
| 6 | 3,89 ± 0,38 | 65,54 ± 7,12 | > 100 | 27,31 ± 4,05 |
| 7 | 4,56 ± 0,61 | 37,99 ± 6,35 | 75,34 ± 6,93 | 30,23 ± 3,66 |
| 8 | 4,94 ± 0,51 | 7,88 ± 1,12 | 12,55 ± 2,13 | 35,47 ± 4,05 |
| 9 | 7,24 ± 0,64 | 12,56 ± 3,05 | 19,87 ± 3,44 | 40,56 ± 3,55 |
| 10 | 8,55 ± 0,59 | 92,56 ± 8,45 | > 100 | 52,38 ± 4,98 |
| 11 | 20,45 ± 3,05 | > 100 | 90,45 ± 8,52 | 85,67 ± 7,34 |
| 12 | 26,78 ± 3,67 | 88,93 ± 7,75 | > 100 | > 100 |

### Beispiel 7: Einfluß von verschiedenen Detergentien und Lösungsmittel auf die Substratstabilität

NAG (0.05 mg/ml) wird in Gegenwart steigender Konzentrationen verschiedener Detergentien und Lösungsmittel für 180 min bei 37°C inkubiert und dann die Extinktionsabnahme bei 550 nm (Auflösung der spez. Substratstruktur) bestimmt (n = 4, mean ± SD).

Ergebnis: Das Substrat (Phospholipid-Vesikel bzw. Micellen) zeigte gegenüber den eingesetzten Agenzien unterschiedliche Empfindlichkeit. Die Extinktion, d.h. die Menge an Substrat verringerte sich in Gegenwart von 1 % DMSO um 10 %, bei Äthanol oder Methanol um max. 20 %, bei 1 % TX-100 oder SDS um max. 30 %. Am effizientesten in der Auflösung der Vesikel/Micellen war DMF, bei 1 % waren über 80 % von NAG aus den Phospholipid/Vesikel Strukturen freigesetzt. Die Rangfolge in der Effizienz der eingesetzten Lösungsmittel und Detergentien in der Auflösung der Substratstruktur ist kompatibel mit einer Verschiebung des Extinktionsmaximum (von 481 nm zu 550 nm) von NAG, bzw. der chromophoren Gruppe (NBD) durch Einbau in die apolare Umgebung von Phospholipidvesikel/-micellen und der dadurch bedingten Entfernung aus wässriger Umgebung. Freisetzung von NAG aus diesen Strukturen in wässriges Milieu durch Auflösung der Vesikel/Micellen (z.B. durch Detergentien) oder Freisetzung der chromophoren Gruppe als NBD-Fettsäure durch lipolytische Spaltung (durch HSL) von NAG führt zu einer Verringerung der Extinktion bei 550 nm und einer Zunahme der Extinktion bei 481 nm. Mit der Stabilität bei 1 % DMSO erfüllt das NAG Substrat eine der Grundvoraussetzungen für einen robusten HTS Assay.

### Absorption bei 550 nm (arb. units)

| Conc. [%] | DMSO | DMF | TX-100 | SDS | Äthanol | Methanol |
|---|---|---|---|---|---|---|
| 0 | 3102 ± 288 | 3204 ± 259 | 3067 ± 284 | 3150 ± 321 | 3096 ± 241 | 3199 ± 280 |
| 0,05 | 3003 ± 259 | 3121 ± 294 | 3166 ± 308 | 3063 ± 288 | 3147 ± 288 | 3056 ± 243 |
| 0,1 | 3199 ± 302 | 2655 ± 308 | 3247 ± 253 | 2935 ± 254 | 3056 ± 213 | 3102 ± 276 |
| 0,2 | 2984 ± 273 | 1952 ± 299 | 3119 ± 277 | 2769 ± 275 | 3097 ± 253 | 2945 ± 241 |
| 0,5 | 3055 ± 289 | 1234 ± 234 | 2954 ± 248 | 2506 ± 301 | 2995 ± 231 | 2683 ± 210 |
| 1 | 2845 ± 254 | 654 ± 112 | 2154 ± 199 | 2296 ± 252 | 2834 ± 256 | 2534 ± 191 |
| 2 | 2317 ± 266 | 251 ± 82 | 956 ± 101 | 1413 ± 178 | 2510 ± 194 | 1985 ± 165 |
| 5 | 1432 ± 187 | 105 ± 42 | 489 ± 77 | 603 ± 89 | 1935 ± 163 | 1438 ± 121 |
| 10 | 372 ± 51 | 38 ± 10 | 167 ± 45 | 102 ± 12 | 873 ± 103 | 475 ± 92 |

### Beispiel 8: Einfluß von verschiedenen Detergentien und Lösungsmittel auf die Aktivität der HSL

NAG (0.05 mg/ml) wird mit HSL (20 µg) in Gegenwart steigender Konzentrationen verschiedener Agenzien für 60 min inkubiert. Die Extinktion bei 481 nm wird bestimmt (n = 4, mean ± SD).
Ergebnis: DMSO bis 1 % reduzierte die Menge an freigesetzter NBD-FA um etwa 10 %. Da bei dieser DMSO-Konzentration bis zu 10 % NAG aus den Phospholipidvesikeln/-micellen durch Auflösung der Strukturen freigesetzt werden, ergibt sich rechnerisch eine Abnahme der Enzymaktivität um 20 %. Bei 0.5 % DMSO beträgt die Reduktion noch 10 %. TX-1 00, Aceton, Äthanol und Methanol bewirken zwischen 0.1 und 1 % eine Erhöhung der HSL-Aktivität, möglicherweise hervorgerufen durch eine effizientere Substratpräsentation. Bei hohen Konzentrationen interferieren sie mit der Aktivität. DMF führt bereits bei Konzentrationen ab 0.1 % zu einem signifikanten Aktivitätsverlust der HSL.

### Absorption bei 481 nm (arb. units)

| Conc. [%] | DMSO | DMF | TX-100 | Aceton | Äthanol | Methanol |
|---|---|---|---|---|---|---|
| 0 | 3534 ± 310 | 3427 ± 213 | 3451 ± 231 | 3325 ± 300 | 3510 ± 329 | 3523 ± 358 |
| 0,05 | 3423 ± 286 | 3365 ± 256 | 3642 ± 296 | 3948 ± 325 | 3776 ± 253 | 3421 ± 290 |
| 0,1 | 3320 ± 319 | 2576 ± 278 | 4164 ± 366 | 4487 ± 376 | 4093 ± 387 | 3341 ± 265 |
| 0,2 | 3301 ± 276 | 1321 ± 154 | 4976 ± 403 | 5876 ± 452 | 4894 ± 325 | 3150 ± 302 |
| 0,5 | 3199 ± 235 | 657 ± 102 | 4065 ± 312 | 6924 ± 537 | 4231 ± 296 | 2317 ± 194 |
| 1 | 3156 ± 196 | 210 ± 78 | 2156 ± 214 | 5421 ± 325 | 3541 ± 294 | 1948 ± 164 |
| 2 | 1488 ± 213 | 134 ± 55 | 1254 ± 143 | 2956 ± 132 | 2143 ± 215 | 1537 ± 78 |
| 5 | 945 ± 169 | 87 ± 32 | 548 ± 77 | 1523 ± 72 | 1327 ± 143 | 932 ± 93 |
| 10 | 272 ± 42 | 14 ± 5 | 315 ± 39 | 505 ± 49 | 423 ± 55 | 675 ± 70 |

### Beispiel 9: Spaltung von NAG durch Lipasen unterschiedlicher Spezifizität

NAG (0.05 mg/ml) wrd mit 20 µg partiell gereinigter HSL, 75 µg partiell gereinigter LPL aus Rattenadipozyten, 20 mU bakterieller Lipase, 50 mU pankreatischer Lipase, 100 mU PLA₂ aus Schlangengift und 0.5 U PC-spezifischer Phospholipase aus

*Bacillus cereus* für 60 min inkubiert. Die Zunahme der Extinktion bei 481 nm wird bestimmt (n = 5, mean ± SD).
Ergebnis: Unter den gegebenen für HSL optimierten Bedingungen zeigten erwartungsgemäß die HSL (100 %) und LPL (etwa 70 %) die größte Aktivität. Die bakterielle und pankreatische Lipase ist deutlich weniger aktiv (25 bzw. 1 %), während die bakterielle PC-spezifische Phospholipase praktisch inaktiv war. Diese stark unterschiedlichen Aktivitäten zeigen die Spezifizität der gewählten Bedingungen des indirekten NAG-Assays für die HSL, so werden z.B. in groben Zellextrakten eventuell enthaltene Phospholipasen nicht erfaßt. Diese Daten zeigen aber auch die prinzipielle Anwendbarkeit des Assayprinzips auf andere Lipasen.

### Zunahme der Absorption bei 481 nm [arb. units]

| Enzym [arb. units] | HSL | LPL | Bacterial lip. | pancreatic lip. | PLA₂ | PLC |
|---|---|---|---|---|---|---|
| 0 | 0 | 0 | 0 | 0 | 0 | 0 |
| 10 | 143 ± 18 | 105 ± 14 | 36 ± 7 | 9 ± 2 | 1 ± 1 | 0 |
| 20 | 321 ± 28 | 187 ± 17 | 81 ± 10 | 14 ± 4 | 3 ± 2 | 1 ± 1 |
| 50 | 744 ± 58 | 489 ± 31 | 148 ± 18 | 39 ± 7 | 5 ± 1 | 2 ± 2 |
| 75 | 1023 ± 95 | 773 ± 56 | 238 ± 29 | 64 ± 10 | 8 ± 2 | 2 ± 1 |
| 100 | 1287 ± 165 | 948 ± 72 | 341 ± 39 | 92 ± 14 | 10 ± 2 | 3 ± 1 |
| 150 | 2106 ± 183 | 1487 ± 132 | 467 ± 57 | 140 ± 18 | 26 ± 6 | 3 ± 1 |
| 200 | 2697 ± 231 | 1952 ± 173 | 748 ± 70 | 188 ± 21 | 35 ± 9 | 5 ± 2 |
| 250 | 3321 ± 349 | 2638 ± 213 | 904 ± 83 | 234 ± 30 | 46 ± 12 | 5 ± 2 |
| 300 | 3855 ± 288 | 2945 ± 250 | 1056 ± 143 | 265 ± 34 | 54 ± 9 | 7 ± 3 |
| 400 | 4427 ± 402 | 3512 ± 288 | 1270 ± 187 | 286 ± 32 | 75 ± 11 | 10 ±3 |

### Beispiel 10: Spaltung verschiedener mit NBD-Fettsäure modifierter Acylglyzeride durch HSL

NAG (0.05 mg/ml) oder verschiedene NBD-Fettsäure-modifizierte Lipide in unterschiedlichen Konzentrationen werden mit 20 µg partiell gereinigter HSL inkubiert. Zu bestimmten Zeitpunkten wird die Extinktion bei 481 nm bestimmt (n = 5, mean ± SD).
Ergebnis:

### Zunahme der Absorption bei 481 nm (arb. units)

| Zeit [min] | NAG 0 | NAG 1 | NAG 2 | NAG 3 | NAG 4 | NAG 5 | NAG 6 |
|---|---|---|---|---|---|---|---|
| 0 | 0 | 0 | 0 | 0 | 0 | 0 | 0 |
| 1 | 39 ± 5 | 33 ± 4 | 13 ± 3 | 6 ± 2 | 2 ± 2 | 2 ± 2 | 1 ± 1 |
| 2 | 67 ± 9 | 59 ± 5 | 28 ± 6 | 15 ± 4 | 3 ± 2 | 5 ± 2 | 2 ± 1 |
| 5 | 158 ± 19 | 149 ± 15 | 69 ± 7 | 34 ± 5 | 5 ± 2 | 13 ± 3 | 2 ± 1 |
| 10 | 378 ± 41 | 341 ± 37 | 132 ± 18 | 61 ± 8 | 8 ± 3 | 28 ± 4 | 2 ± 2 |
| 15 | 561 ± 51 | 502 ± 49 | 234 ± 32 | 106 ± 18 | 13 ± 3 | 47 ± 7 | 3 ± 1 |
| 20 | 789 ± 93 | 711 ± 84 | 358 ± 49 | 149 ± 16 | 21 ± 4 | 63 ± 8 | 3 ± 1 |
| 30 | 1210 ± 193 | 1097 ± 143 | 603 ± 69 | 286 ± 21 | 32 ± 4 | 85 ± 10 | 4 ± 2 |
| 45 | 1784 ± 204 | 1690 ± 173 | 826 ± 55 | 405 ± 53 | 49 ± 5 | 132 ± 16 | 5 ± 2 |
| 60 | 2386 ± 234 | 2207 ± 231 | 1253 ± 186 | 577 ± 61 | 65 ± 9 | 173 ± 19 | 7 ± 3 |
| 75 | 2873 ± 259 | 2714 ± 269 | 1528 ± 162 | 734 ± 83 | 81 ± 11 | 226 ± 29 | 8 ± 3 |
| 90 | 3607 ± 402 | 3396 ± 376 | 1820 ± 197 | 1005 ± 125 | 105 ± 13 | 268 ± 38 | 10 ± 3 |
| 120 | 4312 ± 389 | 4056 ± 411 | 2496 ± 243 | 1384 ± 206 | 148 ± 19 | 325 ± 42 | 21 ± 3 |
| 180 | 4905 ± 423 | 4562 ± 496 | 3045 ± 412 | 1623 ± 188 | 227 ± 31 | 503 ± 51 | 24 ± 5 |

### Beispiel 11: Hemmung der HSL durch Diisopropylphosphofluoridat

NAG (0.05 mg/ml) wird mit 20 µg partiell gereinigter HSL in Gegenwart steigender Konzentrationen and Diisopropylphosphofluoridat für 60 min inkubiert. In Aliquots der Reaktionsansätze wird die Zunahme der Extinktion bei 481 nm bestimmt (indirekter NAG Assay), oder nach Extraktion mit Chloroform/Methanol die Menge an freigesetzter NBD-FA in der organischen Phase durch Fluorimetrie ermittelt (direkter NAG Assay). Alternativ werden Inkubationen der HSL mit [³H]Trioleoylglycerin als Substrat (s.o.) durchgeführt und die Menge freigesetzter radiomarkierter Ölsäure nach Extraktion mit Chloroform/Methanol bestimmt. Die Spaltungsaktivität in Abwesenheit von Inhibitor wird für jeden Assay auf 100 % gesetzt (n = 7, mean ± SD).
Ergebnis: In allen drei Assays ergaben sich typisch sigmoidale Hemmkurven für Diisopropylphosphofluoridat. Die daraus errechneten IC₅₀-Werte unterschieden sich für den indirekten (0.8 mM) oder direkten NAG Assay (1.1 mM) nicht signifikant voneinander. Für die Spaltung von Trioleoylglycerid errechnet sich mit 2.1 mM ein etwas höherer IC₅₀-Wert. Dies steht im Einklang mit den oben festgestellten Unterschieden in den Hemmwirkungen verschiedener Inhibitoren, die mit diesen Assays beobachtet werden (s. Beispiel 6 für mögliche Erklärungen). Unabhängig davon stehen die ermittelten IC₅₀-Werte für die Hemmung der HSL durch Diisopropylphosphofluoridat durch den indirekten NAG Assay in gutem Einklang mit publizierten Daten (P. Stralfors, H. Olsson, P. Belfrage, The Enzymes XVIII, 1987, 147-177; P. Stralfors, P. Belfrage, J. Biol. Chem. 258, 1983, 15146-15151; P. Belfrage, B. Jergil, P. Stralfors, H. Tornquist, FEBS Lett. 75, 1977, 259-263).

### % maximal HSL Aktivität

| Conc. [mM] | NAG-Assay | Freigesetzte NBD-FA | TAG-Assay |
|---|---|---|---|
| 0 | 100 | 100 | 100 |
| 0,01 | 98,9 ± 5,3 | 99,6 ± 4,6 | 99,4 ± 6,2 |
| 0,02 | 96,2 ± 5,1 | 97,4 ± 5,0 | 98,8 ± 5,9 |
| 0,05 | 92,1 ± 4,1 | 94,3 ± 4,4 | 97,5 ± 5,1 |
| 0,1 | 84,3 ± 5,1 | 88,4 ± 5,1 | 95,1 ± 4,5 |
| 0,2 | 74,5 ± 6,3 | 79,2 ± 4,9 | 89,5 ± 5,3 |
| 0,5 | 61,5 ± 5,7 | 67,4 ± 5,3 | 78,9 ± 6,4 |
| 1 | 44,6 ± 4,4 | 53,8 ± 4,9 | 66,3 ± 5,7 |
| 2 | 27,8 ± 3,1 | 38,6 ± 3,4 | 51,9 ± 4,4 |
| 5 | 16,5 ± 2,5 | 24,7 ± 2,8 | 36,7 ± 4,9 |
| 10 | 10,6 ± 1,7 | 13,6 ± 1,9 | 22,6 ± 3,5 |
| 20 | 5,7 ± 1,3 | 7,7 ± 1,5 | 11,8 ± 1,9 |
| 50 | 3,5 ± 1,1 | 4,5 ± 1,2 | 6,4 ± 1,3 |
| 100 | 2,5 ± 0,8 | 2,7 ± 0,6 | 3,1 ± 0,4 |

### Beispiel 12: Durchführbarkeit des indirekten NAG Assays im Mikrotiterplattenformat

NAG wird mit partiell gereinigter HSL in einem Assayvolumen von 200 µl in Wells von 96-Loch-Mikrotiterplatten für unterschiedliche Zeiten inkubiert. Die Extinktion bei 481 nm wird in einem Mikrotiterplatten-Lesegerät ermittelt.
Ergebnis: Unter den gewählten Bedingungen ist die Reaktion bis annähernd 60 min linear. Die Varianz (SD) liegt dabei zwischen 4 und 7 %. Damit ist der indirekte NAG Assay für den Einsatz im HT-Screening geeignet.

### Zunahme der Absorption bei 481 nm [arb. units]

| Zeit [min] | Test: 1 | 2 | 3 | 4 | 5 | 6 | 7 | 8 |
|---|---|---|---|---|---|---|---|---|
| 0 | 0 | 0 | 0 | 0 | 0 | 0 | 0 | 0 |
| 1 | 50 | 48 | 61 | 49 | 56 | 52 | 57 | 49 |
| 2 | 97 | 89 | 107 | 95 | 101 | 116 | 93 | 89 |
| 5 | 238 | 269 | 278 | 243 | 277 | 248 | 235 | 241 |
| 10 | 487 | 534 | 462 | 458 | 552 | 508 | 488 | 460 |
| 15 | 713 | 768 | 709 | 712 | 789 | 780 | 735 | 718 |
| 20 | 978 | 1013 | 967 | 979 | 1097 | 1121 | 1045 | 966 |
| 30 | 1413 | 1579 | 1625 | 1690 | 1523 | 1688 | 1452 | 1410 |
| 45 | 2045 | 2134 | 2239 | 2106 | 2106 | 2238 | 1967 | 2145 |
| 60 | 2367 | 2541 | 2658 | 2755 | 2534 | 2608 | 2536 | 2690 |
| 75 | 2611 | 2894 | 2973 | 3023 | 2871 | 2879 | 2982 | 2895 |
| 90 | 2985 | 3244 | 3310 | 3355 | 3260 | 3122 | 3240 | 3125 |
| 120 | 3127 | 3469 | 3577 | 3601 | 3489 | 3378 | 3507 | 3320 |

### Verwendete Abkürzungen:

- arb. units: arbitrary units
- BSA: Bovine Serum Albumin
- cAMP: zyklisches Adenosinmonophosphat
- DCC: Dicyclohexylcarbodiimid
- DMF: N,N-Dimethylformamid
- DMSO: Dimethylsulfoxid
- DTT: Dithiothreitol
- EDTA: Ethylendiamin-N,N,N',N'-tetraessigsäure
- FAB-MS: Fast atom bombardment mass spectrometry
- HSL: hormon-sensitive Lipase
- Kpᵢ: Kaliumdihydrogenphosphat/Kaliumphosphatpuffer
- LPL: Lipoproteinlipase
- NAG: NBD-Monoacylglycerid: 12-(7-Nitro-benzo[1,2,3]oxadiazol-4-ylamino)-dodecansäure-2,3-dihydroxypropylester
- NBD: 4-Chloro-7-nitrobenzo-2-oxa-1,3-diazol
- NBD-FA: NBD-Fettsäure: 12-(7-Nitro-benzo[1,2,3]oxadiazol-4-ylamino)-dodecansäure
- NIDDM: nicht-insulin-abhängiger Diabetes Mellitus
- PLA₂: Phospholipase A₂
- PLC: Phospholipase C
- PNPB: p-Nitrophenybutyrat
- SD: Standardabweichung
- SDS: Natriumdodecylsulfat
- TAG: [³H]-Trioleoylglycerol
- Tris: Tris(hydroxymethyl)aminomethan
- TLC: Dünnschichtchromatographie
- TX-100: Triton® X-100

## Patentansprüche

1. Verfahren zur Herstellung eines Substrats zur Bestimmung der Integrität komplexer Phospholipid/Lipidstrukturen, **dadurch gekennzeichnet, daß** man
a) eine mit einem Fluoreszenzlabel versehene Fettsäure mit 2,3-Epoxypropanol zu einem Monoacylglycerid in alkoholischer Lösung bei Raumtemperatur unter Zusatz einer Base umsetzt,
b) dieses Monoacylglycerid mit Phospholipiden im Verhältnis von 1:10 bis 10:1 mg/ml einer Ultraschallbehandlung unterzieht, woraus das Substrat resultiert, das an einem Farbumschlag von gelb nach rot erkennbar ist.

2. Verfahren gemäß Anspruch 1, wobei das Fluoreszenzlabel Dansyl oder NBD ist.

3. Verfahren gemäß Anspruch 2, wobei das Fluoreszenzlabel NBD ist.

4. Verfahren gemäß den Ansprüchen 1 bis 3, wobei die Fettsäure eine gesättigte oder ungesättigte Carbonsäure mit einer Kettenlänge von C-8 bis C-20 ist.

5. Verfahren gemäß Anspruch 4, wobei die Fettsäure eine gesättigte C-12-Carbonsäure ist.

6. Verfahren gemäß den Ansprüchen 1 bis 5, wobei als Phospholipide Phosphatidylcholin und Phosphatidylinositol allein oder gemeinsam eingesetzt werden.

7. Verfahren gemäß Anspruch 6, wobei Phosphatidylcholin und Phosphatidylinositol gemeinsam eingesetzt werden im Verhältnis 10 : 1 bis 1 : 10.

8. Verfahren gemäß den Ansprüchen 1 bis 7, wobei das Monoacylglycerid 12-(7-Nitro-benzo[1,2,3]oxadiazol-4-ylamino)-dodecansäure-2,3-dihydroxypropylester ist.

9. Substrat hergestellt nach einem Verfahren der Ansprüche 1 bis 8.

10. Substrat gemäß Anspruch 9 zur Anwendung in einem Verfahren zur Identifizierung von Phospholipid/Lipidstrukturen.

11. Substrat gemäß Anspruch 9 zur Anwendung in einem Verfahren zur Identifizierung von Lipasen und Lipasehemmern.

12. Verfahren zur Herstellung des Monoacylglycerids 12-(7-Nitro-benzo[1,2,3]oxadiazol-4-ylamino)-dodecansäure-2,3-dihydroxypropylester, **dadurch gekennzeichnet, daß** man
12-Aminolaurinsäure zunächst mit 7-Chlor-4-nitrobenzo-2-oxa-1,3-diazol und anschließend das erhaltene Zwischenprodukt mit 2,3-Epoxypropanol in alkoholischer Lösung bei Raumtemperatur unter Zusatz einer Base umsetzt.

13. 12-(7-Nitro-benzo[1,2,3]oxadiazol-4-ylamino)-dodecansäure-2,3-dihydroxypropylester.

14. Verfahren zur Identifizierung von Lipasen und Lipasehemmern, **dadurch gekennzeichnet, daß** man
a) ein Substrat gemäß Anspruch 9 bereitstellt,
b) dieses Substrat mit einer Lipase inkubiert, und
c) die Farbänderung bestimmt.

15. Verfahren zur Bestimmung der Aktivität von Lipasen/Lipasehemmern, **dadurch gekennzeichnet, daß** man
a) ein Substrat gemäß Anspruch 9 bereitstellt,
b) dieses Substrat mit einer Lipase inkubiert,
c) die Geschwindigkeit der Farbänderung bestimmt und
d) die Aktivität ermittelt.

16. Verfahren gemäß Anspruch 15, **dadurch gekennzeichnet, daß** man das Substrat mit einer hormon-sensitiven Lipase inkubiert.

17. Verfahren zur Bestimmung der Detergenzwirkung oder der Cytotoxizität von Verbindungen, **dadurch gekennzeichnet, daß** man
a) ein Substrat gemäß Anspruch 9 bereitstellt,
b) dieses Substrat mit einer Testverbindung inkubiert, und
c) die Farbänderung von rot nach gelb, die cytotoxische Verbindungen und Verbindungen mit Detergenzwirkung kennzeichnen, visuell/optisch oder fluorimetrisch bestimmt.

18. Verfahren zur Bestimmung der Aktivität von Lipidtransportern, **dadurch gekennzeichnet, dass** man
a) ein Substrat gemäß Anspruch 9 bereitstellt,
b) Transporter/Transferproteine in Liposomen funktionell rekonstruiert und
c) die nach b) hergestellten Liposomen zu dem Substrat nach a) gibt und die Farbänderung visuell oder optisch bestimmt.

## Claims

1. A process for the preparation of a substrate for determination of the integrity of complex phospholipid/lipid structures, which comprises
a) reacting a fatty acid provided with a fluorescent label with 2,3-epoxypropanol to give a monoacylglyceride in alcoholic solution at room temperature with addition of a base,
b) subjecting this monoacylglyceride to an ultrasonic treatment with phospholipids in the ratio from 1:10 to 10:1 mg/ml, from which the substrate results, which is recognizable by a color change from yellow to red.

2. The process as claimed in claim 1, where the fluorescent label is dansyl or NBD.

3. The process as claimed in claim 2, where the fluorescent label is NBD.

4. The process as claimed in any of claims 1 to 3, where the fatty acid is a saturated or unsaturated carboxylic acid having a chain length of C-8 to C-20.

5. The process as claimed in claim 4, where the fatty acid is a saturated C-12 carboxylic acid.

6. The process as claimed in any of claims 1 to 5, where the phospholipids employed are phosphatidylcholine and phosphatidylinositol on their own or together.

7. The process as claimed in claim 6, where phosphatidylcholine and phosphatidylinositol are employed together in the ratio 10:1 to 1:10.

8. The process as claimed in any of claims 1 to 7, where the monoacylglyceride is 2,3-dihydroxypropyl 12-(7-nitrobenzo-[1,2,3]oxadiazol-4-ylamino)dodecanoate.

9. A substrate prepared by a process of claims 1 to 8.

10. The substrate as claimed in claim 9 for use in a process for the identification of phospholipid/lipid structures.

11. The substrate as claimed in claim 9 for use in a process for the identification of lipases and lipase inhibitors.

12. A process for the preparation of the monoacylglyceride 2,3-dihydroxypropyl 12-(7-nitrobenzo[1,2,3]oxadiazol-4-ylamino)-dodecanoate, which comprises
reacting 12-aminolauric acid first with 7-chloro-4-nitrobenzo-2-oxa-1,3-diazole and then reacting the intermediate obtained with 2,3-epoxypropanol in alcoholic solution at room temperature with addition of a base.

13. 2,3-Dihydroxypropyl 12-(7-nitrobenzo[1,2,3]oxadiazol-4-ylamino)dodecanoate.

14. A process for the identification of lipases and lipase inhibitors, which comprises
a) preparing a substrate as claimed in claim 9,
b) incubating this substrate with a lipase, and
c) determining the color change.

15. A process for determination of the activity of lipases/lipase inhibitors, which comprises
a) preparing a substrate as claimed in claim 9,
b) incubating this substrate with a lipase,
c) determining the rate of color change and
d) ascertaining the activity.

16. The process as claimed in claim 15, wherein the substrate is incubated with a hormone-sensitive lipase.

17. A process for determination of the detergent action or the cytotoxicity of compounds, which comprises
a) preparing a substrate as claimed in claim 9,
b) incubating this substrate with a test compound, and
c) visually/optically or fluorimetrically determining the color change from red to yellow which identifies cytotoxic compounds and compounds with detergent action.

18. A process for determination of the activity of lipid transporters, which comprises
a) preparing a substrate as claimed in claim 9,
b) functionally reconstructing transporters/transfer proteins in liposomes and
c) adding the liposomes prepared according to b) to the substrate according to a) and determining the color change visually or optically.

## Revendications

1. Procédé pour la préparation d'un substrat destiné à la détermination de l'intégrité de structures de lipides/phospholipides complexes, **caractérisé en ce que**
a) on fait réagir un acide gras, muni d'un marqueur fluorescent, avec du 2,3-époxypropanol, en solution alcoolique, à la température ambiante, avec addition d'une base, pour aboutir à un monoacylglycéride,
b) on soumet à un traitement par ultrasons ce monoacylglycéride avec des phospholipides dans un rapport de 1:10 à 10:1 mg/ml, d'où il résulte le substrat, qui est reconnaissable à un virage du jaune au rouge.

2. Procédé selon la revendication 1, dans lequel le marqueur fluorescent est le groupe dansyle ou NBD.

3. Procédé selon la revendication 2, dans lequel le marqueur fluorescent est le groupe NBD.

4. Procédé selon les revendications 1 à 3, dans lequel l'acide gras est un acide carboxylique saturé ou insaturé ayant une longueur de chaîne de C₈ à C₂₀.

5. Procédé selon la revendication 4, dans lequel l'acide gras est un acide carboxylique saturé en C₁₂.

6. Procédé selon les revendications 1 à 5, dans lequel on utilise en tant que phospholipides de la phosphatidylcholine ou du phosphatidylinositol, seul ou ensemble.

7. Procédé selon la revendication 6, dans lequel on utilise de la phosphatidylcholine et du phosphatidylinositol en un rapport de 10:1 à 1:10.

8. Procédé selon les revendications 1 à 7, dans lequel le monoacylglycéride est le 12-(7-nitro-benzo[1,2,3]oxadiazol-4-ylamino)-dodécanoate de 2,3-dihydroxypropyle.

9. Substrat préparé par un procédé selon les revendications 1 à 8.

10. Substrat selon la revendication 9, pour utilisation dans un procédé destiné à l'identification de structures de lipides/phospholipides.

11. Substrat selon la revendication 9, pour utilisation dans un procédé destiné à l'identification de lipases et d'inhibiteurs de lipases.

12. Procédé pour la préparation du monoacylglycéride 12-(7-nitrobenzo[1,2,3]oxadiazol-4-ylamino)-dodécanoate de 2,3-dihydroxypropyle, **caractérisé en ce qu'**on fait réagir d'abord de l'acide 12-aminolaurique avec du 7-chloro-4-nitrobenzo-2-oxa-1,3-diazole et ensuite le produit intermédiaire obtenu avec du 2,3-époxypropanol en solution alcoolique, à la température ambiante, avec addition d'une base.

13. 12-(7-nitrobenzo[1,2,3]oxadiazol-4-ylamino)-dodécanoate de 2,3-dihydroxypropyle.

14. Procédé pour l'identification de lipases et d'inhibiteurs de lipases, **caractérisé en ce que**
a) on prépare un substrat selon la revendication 9,
b) on met ce substrat à incuber avec une lipase, et
c) on détermine le changement de couleur.

15. Procédé pour la détermination de l'activité de lipases/inhibiteurs de lipases, **caractérisé en ce que**
a) on prépare un substrat selon la revendication 9,
b) on met ce substrat à incuber avec une lipase,
c) on détermine la vitesse du changement de couleur et
d) on détermine l'activité.

16. Procédé selon la revendication 15, **caractérisé en ce qu'**on met le substrat à incuber avec une lipase hormono-sensible.

17. Procédé pour la détermination de l'action détergente ou de la cytotoxicité de composés, **caractérisé en ce que**
a) on prépare un substrat selon la revendication 9,
b) on met ce substrat à incuber avec un composé d'essai, et
c) on détermine visuellement/optiquement ou par fluorimétrie le virage du rouge au jaune, qui caractérise les composés cytotoxiques et les composés à action détergente.

18. Procédé pour la détermination de l'activité de transporteurs de lipides, **caractérisé en ce que**
a) on prépare un substrat selon la revendication 9,
b) on reconstruit de façon fonctionnelle des protéines de transport/transfert dans des liposomes et
c) on ajoute au substrat selon a) les liposomes préparés selon b) et on détermine visuellement ou optiquement le changement de couleur.
